# EUROPEAN PATENT APPLICATION

(11) **EP 4 382 134 A1**
(43) Date of publication of application: **12.06.2024**
(21) Application number: 22853571.2
(22) Date of filing: 05.08.2022
(51) Int. Cl.: A61K 48/00, A61K 31/7088, A61K 47/42, A61P 35/00, C12N 15/113

(54) **PHARMACEUTICAL COMPOSITION FOR PREVENTING OR TREATING TRIPLE-NEGATIVE BREAST CANCER, COMPRISING OLIGONUCLEOTIDE AS ACTIVE INGREDIENT**

(30) Priority: 06.08.2021 KR 20210104105
(71) Applicant: Interoligo Corporation., Anyang-si, Gyeonggi-do 14058 (KR)
(72) Inventor: LEE, Jung Hwan, Yongin-si, Gyeonggi-do 15632 (KR); LEE, Jongook, Seongnam-si, Gyeonggi-do 13457 (KR); LEE, Se Na, Anyang-si, Gyeonggi-do 14016 (KR); PARK, Hanseul, Seoul 07026 (KR)
(74) Representative: Kilger, Ute
(86) International application number: PCT/KR2022/011723
(87) International publication number: WO 2023/014196

(57) **Abstract**

The present disclosure relates to a pharmaceutical composition for preventing or treating triple-negative breast cancer, comprising an oligonucleotide as an active ingredient.

## Description

### TECHNICAL FIELD

The present disclosure relates to a pharmaceutical composition for treating triple-negative breast cancer.

### [Description of National R&D Support]

This application was made with the support of the National Drug Development Project by the National Drug Development Fund, funded by the Ministry of Science and ICT, the Ministry of Trade, Industry and Energy, and the Ministry of Health and Welfare (Proj ect Number: RS-2022-DD128903 formerly HN22C058500).

### BACKGROUND

Breast cancer is the most common cancer among women in advanced countries such as the United States and Europe, becoming the leading cause of death for women between the ages of 40 and 55 in the United States. One in nine women will develop breast cancer in their lifetime, and the number of breast cancer patients is increasing by about 15% each year. In Korea, as of 1995, breast cancer accounted for about 11.9% of female cancer patients, becoming the third most common cancer after cervical and gastric cancer, and the fifth leading cause of cancer death, following stomach, liver, cervical, and lung cancers, with its prevalence increasing annually.

Triple-negative breast cancer (TNBC) accounts for 10-20% of all breast cancer cases. TNBC is defined as tumors that do not express estrogen receptors (ER) and progesterone receptors (PR) and do not overexpress or amplify human epidermal growth factor receptor 2 (HER-2), diagnosed as a subtype of breast cancer tumor through immunohistochemistry. TNBC is characterized by its aggressive clinical behavior and poor prognosis due to rapid resistance to many chemotherapeutic drugs and a lack of suitable targets. Patients with TNBC generally have a worse prognosis compared to those with other types of breast cancer, with higher chances of early recurrence in distant organs and higher mortality rates. Currently, there are no approved targeted therapies available. Traditional microtubule-targeting drugs (MTDs) like paclitaxel and its semi-synthetic derivatives have achieved significant success in the clinical management of breast cancer neoplasms. Anthracycline and taxane chemotherapy are standard treatments for triple-negative breast cancer. However, eventually, most patients with TNBC experience drug resistance, tumor relapse, and/or metastasis after an initial temporary response to the treatment. Therefore, the development of drugs to treat primary or metastatic TNBC with superior efficacy or to effectively and significantly inhibit metastasis from TNBC to other organs (especially the lungs) has been challenging. There is an urgent need to develop innovative and more effective therapeutic approaches that achieve more sustained responses in the treatment of TNBC.

Moreover, it is not easy to completely remove all the micro or residual cancer deeply seated in the surrounding tissues after primary tumor resection during breast cancer surgery. Also, patients typically do not undergo chemotherapy or radiotherapy for 1 to 2 weeks until they recover from the primary cancer surgery. To date, there have been no active treatment methods for residual or micro cancer during this period.

### [Related Document]

### [Patent Literature]

(Patent literature 1) US 2008-0318887 A1

### [Non-Patent Literature]

(Non-patent literature 1) Magdalena M. Dailey et al., Nucleic Acids Research, 2010, Vol. 38, No. 14, pp. 4877-4888
(Non-patent literature 2) YI ZHANG et al. ONCOLOGY LETTERS, 2018, Vol. 15, No. 5, pp. 6233-6240
(Non-patent literature 3) Park et al., Sci. Transl. Med., 2018, Vol. 10, No. 433, eaar1916

### SUMMARY

The present disclosure aims to provide a pharmaceutical composition capable of effectively preventing or treating triple-negative breast cancer (TNBC), a condition that has been difficult to treat with existing methods. The present disclosure presents the world's first method for actively treating TNBC in the post-primary cancer surgery period for which chemotherapy or radiotherapy is difficult to perform. The pharmaceutical composition of the present disclosure is expected to maximize the survival rate of patients with TNBC and reduce the risk of recurrence.

TNBC is characterized by high malignancy and rapid progression and spread, necessitating the development of targeted therapies. However, typical targets for breast cancer targeted therapy, such as the estrogen receptor, HER-2 receptor, and progesterone receptor, are negative (no difference compared to normal cells), leaving no suitable targeted therapy. Especially challenging is the treatment of metastatic cancer following the surgical resection of triple-negative breast cancer, with no existing drugs to prevent or treat such metastasis. Recently, there has been research using a hydrogel patch containing anticancer drugs to study the suppression of cancer metastasis post-surgical resection of TNBC (refer to non-patent literature 3). The study used immunotherapeutic anticancer drugs (anti-PD-1 and anti-CTLA-4), cytokines (IL-15sa), and small molecule drugs (lenalidomide, celecoxib, STING-RR, and resiquimod). In experiments conducted on mice, the most successful group (resiquimod and STING-RR group) showed a 60% survival rate after 50 days post-insertion of the drug/hydrogel patch at the resection site. This result represents the most advanced result in implantable target therapy for inhibiting metastasis of TNBC to date. In contrast, the pharmaceutical composition according to an embodiment of the present disclosure showed an astonishing 100% survival rate, indicating a very effective treatment that has not been seen in preclinical animal experiments to date.

The present disclosure aims to provide a pharmaceutical composition including an oligonucleotide as an active ingredient for the treatment of TNBC. The present disclosure is also to provide a pharmaceutical composition with excellent stability and anticancer effects in vivo.

The present disclosure is intended to provide a pharmaceutical composition that demonstrates excellent effectiveness in treating primary or metastatic TNBC, which has been difficult to treat with existing therapies.
1. An embodiment of the present disclosure provides a pharmaceutical composition for preventing or treating triple-negative breast cancer (TNBC), comprising a modified oligonucleotide having a structure represented by Chemical Formula 1:

   [Chemical Formula 1] (N)ₓ-[TGG]ₘ[TTG][TGG]ₙ-(M)_{y}

   or a pharmaceutically acceptable salt thereof
   wherein N and M are represent deoxyuridine (dU), deoxycytidine (dC), uridine (U), or cytidine (C) bound with a halogen or hydroxyl at the 5-position or 2'-position; x and y are independently integers from 0 to 10, provided that x and y are not both 0; n is an integer from 1 to 10; and m is an integer from 1 to 10).
2. In an embodiment, N and M may independently be selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluorodeoxycytidine, capecitabine, and bromovinyldeoxyuridine.
3. In an embodiment, the modified oligonucleotide may have a structure represented by any one of Chemical Formulas 2 to 34:

   [Chemical Formula 2] (N)₂-[TGG]₁[TTG][TGG]₁,

   [Chemical Formula 3] (N)₂-[TGG]₁[TTG][TGG]₂,

   [Chemical Formula 4] (N)₂-[TGG]₂[TTG][TGG]₁,

   [Chemical Formula 5] (N)₂-[TGG]₂[TTG][TGG]₂,

   [Chemical Formula 6] (N)₂-[TGG]₂[TTG][TGG]₃,

   [Chemical Formula 7] (N)₂-[TGG]₃[TTG][TGG]₂,

   [Chemical Formula 8] (N)₂-[TGG]₃[TTG][TGG]₃,

   [Chemical Formula 9] (N)₂-[TGG]₃[TTG][TGG]₄,

   [Chemical Formula 10] (N)₂-[TGG]₄[TTG][TGG]₃,

   [Chemical Formula 11] (N)₂-[TGG]₄[TTG][TGG]₄,

   [Chemical Formula 12] (N)₂-[TGG]₄[TTG][TGG]₅,

   [Chemical Formula 13] (N)₂-[TGG]₅[TTG][TGG]₄,

   [Chemical Formula 14] (N)₂-[TGG]₅[TTG][TGG]₅,

   [Chemical Formula 15] (N)₂-[TGG]₅[TTG][TGG]₆,

   [Chemical Formula 16] (N)₂-[TGG]₆[TTG][TGG]₅,

   [Chemical Formula 17] (N)₂-[TGG]₆[TTG][TGG]₆,

   [Chemical Formula 18] [TGG]₄[TTG][TGG]₄-(M)₁,

   [Chemical Formula 19] [TGG]₄[TTG][TGG]₄-(M)₂,

   [Chemical Formula 20] [TGG]₄[TTG][TGG]₄-(M)₃,

   [Chemical Formula 21] [TGG]₄[TTG][TGG]₄-(M)₄,

   [Chemical Formula 22] [TGG]₄[TTG][TGG]₄-(M)₅,

   [Chemical Formula 23] [TGG]₄[TTG][TGG]₅-(M)₁,

   [Chemical Formula 24] [TGG]₄[TTG][TGG]₅-(M)₂,

   [Chemical Formula 25] [TGG]₄[TTG][TGG]₅-(M)₃,

   [Chemical Formula 26] [TGG]₄[TTG][TGG]₅-(M)₄,

   [Chemical Formula 27] [TGG]₄[TTG][TGG]₅-(M)₅,

   [Chemical Formula 28] [TGG]₄[TTG][TGG]₄-(M)₁₀,

   [Chemical Formula 29] (N)₁-[TGG]₄[TTG][TGG]₄-(M)₁,

   [Chemical Formula 30] (N)₃-[TGG]₄[TTG][TGG]₄-(M)₃,

   [Chemical Formula 31] (N)₅-[TGG]₄[TTG][TGG]₄-(M)₅,

   [Chemical Formula 32] (N)₁-[TGG]₄[TTG][TGG]₅-(M)₁,

   [Chemical Formula 33] (N)₃-[TGG]₄[TTG][TGG]₅-(M)₃,

   [Chemical Formula 34] (N)₅-[TGG]₄[TTG][TGG]₅-(M)₅
4. In an embodiment, n may be an integer from 1 to 5, and m may be an integer from 1 to 5.
5. In an embodiment, x and y may independently be integers from 0 to 5, provided that x and y are not both 0.
6. In an embodiment, the modified oligonucleotide may have the structure represented by Chemical Formula 12:

   [Chemical Formula 12] (N)₂-[TGG]₄[TTG][TGG]₅.
7. In an embodiment, N may be 2',2'-difluorodeoxycytidine.
8. In an embodiment, the TNBC may be metastatic triple-negative breast cancer.
9. In an embodiment, the pharmaceutical composition may be administered to a subject who has undergone surgical resection of TNBC.
10. In an embodiment, the pharmaceutical composition may prevent or treat metastatic cancer or tumors in the subject.
11. In an embodiment, the metastatic cancer may be lung cancer, brain cancer, osteosarcoma, liver cancer, or lymphoma.
12. In an embodiment, the modified oligonucleotide may be administered at a dose of about 0.01 mg/kg to about 100 mg/kg.
13. In an embodiment, the pharmaceutical composition may be administered once a week, once every two weeks, once every three weeks, or once a month.
14. In an embodiment, the pharmaceutical composition may further comprise a biomaterial.
15. In an embodiment, the pharmaceutical composition may be administered in a formulation of a collagen dispersion or a hydrogel dispersion.
16. In an embodiment, the pharmaceutical composition may be administered in a formulation of a sol-gel or patch form.
17. In an embodiment, the pharmaceutical composition may be implanted at a site of surgical resection of TNBC in a subject in need thereof.
18. In an embodiment, the pharmaceutical composition may be administered intravenously, intramuscularly, intra-arterially, intraperitoneally, intranasally, intravaginally, intravesically, intradermally, transdermally, topically, or subcutaneously to a subject in need thereof.
19. An embodiment of the present disclosure may contemplate a pharmaceutical composition for preventing or treating metastatic cancer or tumors occuring after the surgical resection of TNBC, comprising a modified oligonucleotide having a structure represented by Chemical Formula 1:

   [Chemical Formula 1] (N)ₓ-[TGG]ₘ[TTG][TGG]ₙ-(M)_{y}

   or a pharmaceutically acceptable salt thereof, wherein the pharmaceutical composition is administered to a subject who has undergone surgical resection of TNBC,
   wherein N and M independently are deoxyuridine (dU), deoxycytidine (dC), uridine (U), or cytidine (C) bound with a halogen or hydroxyl at the 5-position or 2'-position; x and y are independently integers from 0 to 10, provided that x and y are not both 0; n is an integer from 1 to 10; and m is an integer from 1 to 10).

The modified oligonucleotide according to an embodiment of the present disclosure may effectively target nucleolin that exists on the surface of cancer cells, in the cytoplasm, or in the nucleus.

The modified oligonucleotide according to an embodiment of the present disclosure may inhibit the growth of cancer cells or kill cancer cells.

The modified oligonucleotide in which a modified nucleic acid (N) is bound to an oligonucleotide in a specific sequence, according to an embodiment of the present disclosure, may reduce the rate of enzymatic degradation in vivo.

The pharmaceutical composition according to an embodiment of the present disclosure may effectively prevent or treat triple-negative breast cancer.

The pharmaceutical composition according to an embodiment of the present disclosure can effectively prevent or treat metastatic cancer or tumors that occur after the surgical resection of triple-negative breast cancer. The pharmaceutical composition according to an embodiment of the present disclosure may effectively inhibit the metastasis of cancer or tumors after the surgical resection of triple-negative breast cancer.

The pharmaceutical composition according to an embodiment of the present disclosure may be administered immediately after the surgical resection of triple-negative breast cancer tumor, allowing active and effective treatment of triple-negative breast cancer in a post-primary cancer surgery period for which chemotherapy or radiotherapy is difficult to perform.

### BRIEF DESCRIPTION OF DRAWINGS

FIG. 1 is a graph showing results of inhibition of cell growth for gemcitabine, IO101L, and IO101L-control on 4T1 cell line.
FIG. 2 is a graph showing results of inhibition of cell growth for gemcitabine, IO101L, and IO101L-control on MDA-MB-231 cell line.
FIG. 3 is a graph showing results of inhibition of cell growth gemcitabine, IO101L, and IO101L-control on MCF-7 cell line.
FIG. 4 is a graph showing experimental results of cellular uptake in 4T1 cells.
FIG. 5 shows IVIS images before and after resection of primary tumors, illustrating a mouse model construction process.
FIG. 6 is a diagram illustrating the construction of a triple-negative breast cancer orthotopic animal model and the administration schedule of the test substance.
FIGS. 7a to 7d are in vivo imaging system (IVIS) images of triple-negative breast cancer orthotopic animal models treated with no drugs and intraperitoneally injected with test substances (gemcitabine, IO101L-control, and IO101L).
FIG. 8 is a plot showing the survival rates of triple-negative breast cancer orthotopic animal model treated with no drugs and intraperitoneally injected with test substances (gemcitabine, IO101L-control, and IO101L).
FIGS. 9a and 9b are IVIS images of triple-negative breast cancer orthotopic animal models treated with no drugs and a 2mg IO101L-loaded collagen patch implanted at the tumor removal site.
FIG. 10 is a plot showing the survival rates of triple-negative breast cancer orthotopic animal model treated with no drugs and a 2mg IO101L-loaded collagen patch implanted at the tumor removal site.
FIG. 11 is a plot showing body weight changes of triple-negative breast cancer orthotopic animal models treated with no drugs and a 2mg IO101L-loaded collagen patch implanted at the tumor removal site.
FIGS. 12a to 12f are IVIS images of triple-negative breast cancer orthotopic animal models treated with no drugs, single collagen patches, gemcitabine-loaded collagen patches, and IO101L-loaded collagen patches with different doses implanted at the tumor removal site.
FIG. 13 is a plot showing the survival rates of triple-negative breast cancer orthotopic animal models treated with no drugs, single collagen patches, gemcitabine-loaded collagen patches, and IO101L-loaded collagen patches with different doses implanted at the tumor removal site.
FIG. 14 is a plot showing body weight changes of triple-negative breast cancer orthotopic animal models treated with no drugs, single collagen patches, gemcitabine-loaded collagen patches, and IO101L-loaded collagen patches with different doses implanted at the tumor removal site.
FIG. 15 is a diagram illustrating the process of constructing a triple-negative breast cancer orthotopic animal model and the method of administering the test substance.
FIGS. 16a and 16b are IVIS images of triple-negative breast cancer orthotopic animal models treated with no drugs and an IO101L-loaded hydrogel patch implanted at the tumor removal site.

### DETAILED DESCRIPTION

The various embodiments described herein are exemplified for the purpose of clearly describing the technical idea of the present disclosure, and are not intended to limit the technical idea of the present disclosure to specific embodiments. The technical idea of the present disclosure includes various modifications, equivalents, alternatives of each of the embodiments described in this document, and embodiments selectively combined from all or part of the respective embodiments.

The terms used herein, including technical or scientific terms, may have meanings that are generally understood by a person having ordinary knowledge in the art to which the present disclosure pertains, unless otherwise specified.

A singular expression used herein can include a meaning of plurality, unless otherwise mentioned, and the same is applied to a singular expression stated in the claims.

The term "about" or "approximately" used herein may refer to the usual error range for each value, as is widely known to a person skilled in the art. In the context of a numerical value or range described herein, the term may mean±20%, ±15%, ±10%, ±9%, ±8%, ±7%, ±6%, ±5%, ±4%, ±3%, ±2%, or ±1% of a numerical value or range recited or claimed in one embodiment.

As used herein, the expressions such as "comprise," "may comprise," "provided with," "may be provided with," "have," and "may have" should be understood as open-ended terms that imply the possibility of including other embodiments unless stated otherwise in the phrases or sentences including the expressions.

As used herein, the term "and/or" includes any and all combinations of one or more of the associated listed items.

The embodiments described in the present disclosure are understood to include those that "comprise", are "consisting of", and/or are "consisting essentially of" the disclosed embodiments.

The term "pharmaceutical composition" refers to a preparation which is in such form as to permit the biological activity of an active ingredient contained therein to be effective, and which contains no additional components which are unacceptably toxic to a subject to which the composition would be administered.

As used herein, the term "pharmaceutically acceptable" means that, which is useful in preparing a pharmaceutical composition that is generally safe, non-toxic, and neither biologically nor otherwise undesirable and includes that which is acceptable for veterinary as well as human pharmaceutical use and, for example, may mean those approved by the management agency of each country or listed in the pharmacopoeia of each country. As used herein, the term "pharmaceutically acceptable salt" may refer to a product that contains an ionic bond and is conventionally produced by reacting a compound with an acid or base suitable for administration of a compound to a subject.

A "pharmaceutically acceptable excipient" refers to an ingredient, other than an active ingredient, which is nontoxic to a subject. A pharmaceutically acceptable excipient includes, but is not limited to, a binder, a filter, a solvent, a buffer, a toxic agent, a stabilizer, an antioxidant, a surfactant, a lubricant, etc.

As used herein, the term "pharmaceutically acceptable carrier" refers to a component in a pharmaceutical composition, other than the active ingredient, which is non-toxic to the recipient. A pharmaceutically acceptable carrier may include buffers, excipients, stabilizers, or preservatives, but with no limitations thereto. The term "carriers," as used herein, may include pharmaceutically acceptable excipients, adjuvants, or stabilizers that are non-toxic to cells or mammals at the dosages and concentrations employed. Often, the pharmaceutically acceptable carrier is a water-soluble pH buffer solution. Examples of pharmaceutically acceptable carriers include buffers such as phosphates, citrates, and other organic acids; antioxidants including ascorbic acid; low molecular weight (less than about ten residues) polypeptides; proteins such as serum albumin, gelatin, or immunoglobulins; hydrophilic polymers such as polyvinylpyrrolidone; amino acids such as glycine, glutamine, asparagine, arginine, or lysine; monosaccharides, disaccharides, and other carbohydrates including glucose, mannose, or dextrins; chelating agents such as EDTA; sugar alcohols such as mannitol or sorbitol; salts forming counter ions such as sodium; and/or nonionic surfactants such as TWEEN^{®}, polyethylene glycol (PEG), and PLURONICS^{®}, but are not limited thereto.

The term "pharmaceutically acceptable carrier and/or diluent", as used herein, encompasses all solvents, dispersion media, coatings, antimicrobial and antifungal agents, isotonic and absorption delaying agents, and the like, but are not limited thereto.

The term "effective amount," as used herein, refers to an amount sufficient to elicit the desired clinical or biochemical response. The effective amount can be administered in a single dose or multiple doses. It may mean an amount sufficient to alleviate, improve, stabilize, slow down, or delay progression of a disease state.

In this disclosure, the terms "pharmacologically effective amount," "dosage," "therapeutic effective amount," or "effective dose" of a pharmaceutical composition refers to an amount effective, at dosages and for periods necessary, to achieve the desired therapeutic or prophylactic outcome.

The term "subject" or "patient," as used herein, refers to mammals, which include livestock (e.g., cattle, sheep, cats, dogs, and horses), primates (e.g., humans and non-human primates such as monkeys), rabbits, and rodents (e.g., mice and rats), but with no limitations thereto. In an embodiment, the subject or patient may be a human.

The term "treatment," as used herein, means an approach to obtain a beneficial or desired clinical result. For the purposes of the present disclosure, beneficial or desired clinical results include, but are not limited to, alleviation of detectable or undetectable symptoms, reduction in severity of the disease, stabilization of the disease state (i.e., not deteriorating), delaying or slowing of disease progression, improvement or alleviation of the disease state, and partial or complete remission of the disease. "Treatment" may also mean extending survival as compared to expected life span in the absence of treatment. "Treatment" encompasses both actions for therapy and measures for prevention or prophylaxis. The subjects needing treatment may include those already with the disease as well as those in which the disease is to be prevented. The term "palliating a symptom" in a disease, as used herein, means that the degree of disease state and/or unwanted clinical symptoms are reduced, and/or the time course of the progression of the disease is slowed or lengthened, compared to a situation without treatment.

The term "preventing" or "to prevent," as used herein, means (i) reducing the risk of developing one or more clinical symptoms of a developed or undeveloped disease in a subject who is exposed or potentially susceptible to the disease but has not yet experienced or manifested symptoms of the disease, or (ii) reducing the risk of acquiring the disease.

An embodiment of the present disclosure is related to a pharmaceutical composition comprising a modified oligonucleotide or a pharmaceutically acceptable salt thereof for treating triple-negative breast cancer.

An embodiment of the present disclosure is related to a modified oligonucleotide.

In an embodiment, the modified oligonucleotide may be a conjugate of an oligonucleotide and a modified nucleic acid.

In an embodiment, the oligonucleotide may comprise or consist of the nucleic acid sequence [TGG]ₘ[TTG][TGG]ₙ.

In an embodiment, n may be an integer from 1 to 10, from 1 to 9, from 1 to 8, from 1 to 7, from 1 to 6, from 1 to 5, from 1 to 4, from 1 to 3, or from 1 to 2. Additionally, n may be an integer from 1 to 10, 2 to 9, 3 to 8, 4 to 7, or 5 to 6.

In an embodiment, m may be an integer from 1 to 10, from 1 to 9, from 1 to 8, from 1 to 7, from 1 to 6, from 1 to 5, from 1 to 4, from 1 to 3, or from 1 to 2. In addition, m may be an integer from 1 to 10, from 2 to 9, from 3 to 8, from 4 to 7, or from 5 to 6.

In an embodiment, the oligonucleotide may be any one of the nucleic acid sequences listed in Table 1 below, but is not limited thereto.

**TABLE 1**

| SEQ ID NO: | Oligonucleotide ([TGG]ₘ[TTG][TGG]ₙ)(5' → 3') |
|---|---|
| 1 | [TGG]₁[TTG][TGG]₁ |
| 2 | [TGG]₁[TTG][TGG]₂ |
| 3 | [TGG]₂[TTG][TGG]₁ |
| 4 | [TGG]₂[TTG][TGG]₂ |
| 5 | [TGG]₂[TTG][TGG]₃ |
| 6 | [TGG]₃[TTG][TGG]₂ |
| 7 | [TGG]₃[TTG][TGG]₃ |
| 8 | [TGG]₃[TTG][TGG]₄ |
| 9 | [TGG]₄[TTG][TGG]₃ |
| 10 | [TGG]₄[TTG][TGG]₄ |
| 11 | [TGG]₄[TTG][TGG]₅ |
| 12 | [TGG]₅[TTG][TGG]₄ |
| 13 | [TGG]₅[TTG][TGG]₅ |
| 14 | [TGG]₅[TTG][TGG]₆ |
| 15 | [TGG]₆[TTG][TGG]₅ |
| 16 | [TGG]₆[TTG][TGG]₆ |
| 17 | [TGG]₆[TTG][TGG]₇ |
| 18 | [TGG]₇[TTG][TGG]₆ |
| 19 | [TGG]₇[TTG][TGG]₇ |
| 20 | [TGG]₇[TTG][TGG]₈ |
| 21 | [TGG]₈[TTG][TGG]₇ |
| 22 | [TGG]₈[TTG][TGG]₈ |
| 23 | [TGG]₈[TTG][TGG]₉ |
| 24 | [TGG]₉[TTG][TGG]₈ |
| 25 | [TGG]₉[TTG][TGG]₉ |
| 26 | [TGG]₉[TTG][TGG]₁₀ |
| 27 | [TGG]₁₀[TTG][TGG]₉ |
| 28 | [TGG]₁₀[TTG][TGG]₁₀ |

In an embodiment, the oligonucleotide may be rich in guanosine to form a G-quadruplex structure and may be an aptamer specific for nucleolin.

As used herein, the term "nucleolin" refers to a protein that is expressed at high levels in transformed cells. Most tumor cells not only express nucleolin in the cytoplasm and nucleus but are also known to expose nucleolin on the cell surface. Nucleolin performs various functions in cells and may be involved in ribosome production, cell growth, and DNA replication.

In an embodiment, the oligonucleotide may bind more selectively to cancer cells and inhibit cancer cell growth through various mechanisms within the cell.

In an embodiment, the modified oligonucleotide may result from bonding one or more modified nucleic acids to an oligonucleotide. Such a bond can allow the modified nucleic acid to be stabilized or prevented from being deactivated in vivo.

In an embodiment, the modified nucleic acid may not be a naturally occurring nucleic acid or nucleotide, but a deoxyuridine (dU), deoxycytidine (dC), uridine (U), or cytidine (C) bound with at least one halogen or hydroxyl at the 5-position or 2'-position. For example, the modified nucleic acid may be selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluorodeoxycytidine, capecitabine, bromovinyldeoxyuridine, or derivatives thereof, but is not limited to these.

In an embodiment, the modified nucleic acid may be independently bonded or conjugated to any position of the oligonucleotide sequence. For example, the modified nucleic acid may be bonded or conjugated to the 5'-position, 2'-position, or any position within the oligonucleotide sequence.

In an embodiment, the modified nucleic acid may be connected to at least one nucleotide located in the 5' direction or 3' direction or within the oligonucleotide sequence by a linker.

The term "modified nucleic acid", as used in the present disclosure, may also mean analogs, substitutes, or esters of naturally occurring nucleotides (A, G, T/U, and C) and encompasses a wide concept of modified nucleotides that are readily available to those skilled in the art. For example, modified nucleotides may mean nucleotides with substitution at the C-5 position (e.g., C-5 modified pyrimidine) or nucleotides whose sugar (ribose or deoxyribose) is modified, or nucleotides with both such modifications.

In an embodiment, the linker may be [-(CH₂)ₐ-], [-(CH₂CH₂O)_{b}-], [butylramidomethyl-1-(2-nitrophenyl)-ethyl]-2-cyanoethyl-], [1', 2'-dideoxyribose-], or (PEG)_{y}. In an embodiment, a may be an integer from 1 to 10, from 2 to 9, from 3 to 8, from 4 to 7, from 5 to 6; b may be an integer from 1 to 10, from 2 to 9, from 3 to 8, from 4 to 7, from 5 to 6; and y may be an integer from 1 to 20, from 2 to 19, from 3 to 18, from 4 to 17, from 5 to 16, from 6 to 15, from 7 to 14, from 8 to 13, from 9 to 12, or from 10 to 11.

In an embodiment, when the linker is combined, the modified oligonucleotide may be expressed as having, for example, the structure of (N)ₓ-L-[TGG]ₘ[TTG][TGG]ₙ-L-(M)_{y} [L = linker].

In an embodiment, when the modified nucleic acid is connected to the 3' direction of the oligonucleotide, additional idT (inverted dT), LNA (locked nucleic acid), polyethylene glycol (PEG), or 2'OMeNu (2'-methoxy nucleosides) can be further bonded to the 3' direction of the modified nucleic acid.

In an embodiment, the 3' end of the modified oligonucleotide can be protected from the attack of nucleases by further bonding idT, LNA, PEG, or 2'OMeNu to the 3' end of the modified nucleic acid, whereby the degradation rate of the modified oligonucleotide in vivo is reduced to allow the modified nucleic acid to be bound to the oligonucleotide for a longer period, thereby potentially increasing the anticancer efficacy of the modified nucleic acid.

In an embodiment, the modified oligonucleotide may have the structure represented by Chemical Formula 1:

[Chemical Formula 1] (N)ₓ-[TGG]ₘ[TTG][TGG]ₙ-(M)_{y}

wherein, N and M are modified nucleic acids, and N and M may be the same or different types of modified nucleic acids. The details of modified nucleic acids are as previously described.

In an embodiment, in Chemical Formula 1, x and y each may be independently an integer from 0 to 10, provided that x and y are not both 0. In an embodiment, in Chemical Formula 1, x may be an integer from 0 to 10, from 1 to 9, from 2 to 8, from 3 to 7, from 4 to 6, and y may be an integer from 0 to 10, from 1 to 9, from 2 to 8, from 3 to 7, from 4 to 6, provided that x and y are not both.

In an embodiment, in Chemical Formula 1, n may be an integer from 1 to 10, and m may be an integer from 1 to 10. In an embodiment, in Chemical Formula 1, n may be an integer from 1 to 10, from 1 to 9, from 1 to 8, from 1 to 7, from 1 to 6, from 1 to 5, from 1 to 4, from 1 to 3, or from 1 to 2. Additionally, in Chemical Formula 1, n may be an integer from 1 to 10, from 2 to 9, from 3 to 8, from 4 to 7, from 5 to 6.

In an embodiment, in Chemical Formula 1, m may be an integer from 1 to 10, from 1 to 9, from 1 to 8, from 1 to 7, from 1 to 6, from 1 to 5, from 1 to 4, from 1 to 3, or from 1 to 2. In another embodiment, in Chemical Formula 1, m may be from 1 to 10, from 2 to 9, from 3 to 8, from 4 to 7, or from 5 to 6.

In an embodiment, the modified oligonucleotide may have the structure of any one of the chemical formulas listed in Table 2, below.

**TABLE 2**

| Drug (N) type | Modified oligonucleotide ((N)ₓ-[TGG]ₘ[TTG][TGG]ₙ) |
|---|---|
| 2',2'-difluorodeoxycytidine | (N)₁-[TGG]₄[TTG][TGG]₄ |
| | (N)₂-[TGG]₄[TTG][TGG]₄ |
| | (N)₃-[TGG]₄[TTG][TGG]₄ |
| | (N)₄-[TGG]₄[TTG][TGG]₄ |
| | (N)₅-[TGG]₄[TTG][TGG]₄ |
| | (N)₁-[TGG]₄[TTG][TGG]₅ |
| | (N)₂-[TGG]₄[TTG][TGG]₅ |
| | (N)₃-[TGG]₄[TTG][TGG]₅ |
| | (N)₄-[TGG]₄[TTG][TGG]₅ |
| | (N)₅-[TGG]₄[TTG][TGG]₅ |
| 5-fluoro-deoxyuridine | (N)₁-[TGG]₄[TTG][TGG]₄ |
| | (N)₅-[TGG]₄[TTG][TGG]₄ |
| | (N)₁₀-[TGG]₄[TTG][TGG]₄ |
| | (N)₁-[TGG]₄[TTG][TGG]₅ |
| | (N)₅-[TGG]₄[TTG][TGG]₅ |
| | (N)₁₀-[TGG]₄[TTG][TGG]₅ |
| cytosine arabinoside | (N)₁-[TGG]₄[TTG][TGG]₄ |
| | (N)₂-[TGG]₄[TTG][TGG]₄ |
| | (N)₃-[TGG]₄[TTG][TGG]₄ |
| | (N)₁-[TGG]₄[TTG][TGG]₅ |
| | (N)₂-[TGG]₄[TTG][TGG]₅ |
| | (N)₃-[TGG]₄[TTG][TGG]₅ |

| Drug (N) type | Modified oligonucleotide ([TGG]ₘ[TTG][TGG]ₙ₋(N)ₓ) |
|---|---|
| 2',2'-difluorodeoxycytidine | [TGG]₄[TTG][TGG]₄-(N)₁ |
| | [TGG]₄[TTG][TGG]₄-(N)₂ |
| | [TGG]₄[TTG][TGG]₄-(N)₃ |
| | [TGG]₄[TTG][TGG]₄-(N)₄ |
| | [TGG]₄[TTG][TGG]₄-(N)₅ |
| | [TGG]₄[TTG][TGG]₅-(N)₁ |
| | [TGG]₄[TTG][TGG]₅-(N)₂ |
| | [TGG]₄[TTG][TGG]₅-(N)₃ |
| | [TGG]₄[TTG][TGG]₅-(N)₄ |
| | [TGG]₄[TTG][TGG]₅-(N)₅ |
| 5-fluoro-deoxyuridine | [TGG]₄[TTG][TGG]₄-(N)₁ |
| | [TGG]₄[TTG][TGG]₄-(N)₅ |
| | [TGG]₄[TTG][TGG]₄-(N)₁₀ |
| | [TGG]₄[TTG][TGG]₅-(N)₁ |
| | [TGG]₄[TTG][TGG]₅-(N)₅ |
| | [TGG]₄[TTG][TGG]₅-(N)₁₀ |
| cytosine arabinoside | [TGG]₄[TTG][TGG]₄-(N)₁ |
| | [TGG]₄[TTG][TGG]₄-(N)₂ |
| | [TGG]₄[TTG][TGG]₄-(N)₃ |
| | [TGG]₄[TTG][TGG]₅-(N)₁ |
| | [TGG]₄[TTG][TGG]₅-(N)₂ |
| | [TGG]₄[TTG][TGG]₅-(N)₃ |

| Drug (N) type | Modified oligonucleotide ((N)ₓ-[TGG]ₘ[TTG][TGG]ₙ-(N)_{y}) |
|---|---|
| 2',2'-difluorodeoxycytidine | (N)₁-[TGG]₄[TTG][TGG]₄-(N)₁ |
| | (N)₃-[TGG]₄[TTG][TGG]₄-(N)₃ |
| | (N)₅-[TGG]₄[TTG][TGG]₄-(N)₅ |
| | (N)₁-[TGG]₄[TTG][TGG]₅-(N)₁ |
| | (N)₃-[TGG]₄[TTG][TGG]₅-(N)₃ |
| | (N)₅-[TGG]₄[TTG][TGG]₅-(N)₅ |
| 5-fluoro-deoxyuridine | (N)₁-[TGG]₄[TTG][TGG]₄-(N)₁ |
| | (N)₃-[TGG]₄[TTG][TGG]₄-(N)₃ |
| | (N)₅-[TGG]₄[TTG][TGG]₄-(N)₅ |
| | (N)₁-[TGG]₄[TTG][TGG]₅-(N)₁ |
| | (N)₃-[TGG]₄[TTG][TGG]₅-(N)₃ |
| | (N)₅-[TGG]₄[TTG][TGG]₅-(N)₅ |
| cytosine arabinoside | (N)₁-[TGG]₄[TTG][TGG]₄-(N)₁ |
| | (N)₃-[TGG]₄[TTGI[TGG]₄-(N)₃ |
| | (N)₅-[TGG]₄[TTG][TGG]₄-(N)₅ |
| | (N)₁-[TGG]₄[TTG][TGG]₅-(N)₁ |
| | (N)₃-[TGG]₄[TTG][TGG]₅-(N)₃ |
| | (N)₅-[TGG]₄[TTG][TGG]₅-(N)₅ |

In an embodiment, the modified oligonucleotide may result from linking at least one of gemcitabine (2',2'-difluorodeoxycytidine) to the 5' or 3' direction of the oligonucleotide of SEQ ID NO: 10. For example, the modified oligonucleotide may have up to 10, 9, 8, 7, 6, 5, 4, 3, or 2 gemcitabine molecules bound in the 5' direction to the oligonucleotide of SEQ ID NO: 10.

In an embodiment, the modified oligonucleotide may result from linking at least one of gemcitabine (2',2'-difluorodeoxycytidine) to the 5' or 3' diction of the oligonucleotide of SEQ ID NO: 11. For example, the modified oligonucleotide may have up to 10, 9, 8, 7, 6, 5, 4, 3, or 2 gemcitabine molecules bound in the 5' direction to the oligonucleotide of SEQ ID NO: 11.

In an embodiment, a method for linking the oligonucleotide and the modified nucleic acid may comprise the step of bonding the modified nucleic acid to at least one of the 5' or 3' ends of the aforementioned oligonucleotide.

In an embodiment, another method for connecting the oligonucleotide and modified nucleic acid may comprise chemically synthesizing a linker to at least one of the 5' or 3' ends of the aforementioned oligonucleotide, followed by bonding the modified nucleic acid to the connected linker.

In an embodiment, the step of binding the modified nucleic acid to manufacture the modified oligonucleotide may include using a solid-phase reactor.

In an embodiment, the oligonucleotide is a GRO (guanine-rich oligonucleotide) aptamer, and such oligonucleotides can be differently denoted depending on the linker present therein. For instance, according to U.S. Patent No. 2008/0318887 A, the oligonucleotide may have the structure [GGT]ₘ-TGT-[GGT]ₙ-GG, and according to a non-patent literature 1 [Nucleic Acids Research, 2010, 38(14), 4877-4888], the oligonucleotide may have the structure [GGT]ₘ-GG-TTGT-[GGT]ₙ-GG. In an embodiment, the oligonucleotide may be bound with modified nucleic acid as described in the foregoing, and the nucleic acids that are constituents of the oligonucleotide may be substituted with modified nucleotides. In an embodiment, the oligonucleotide may be represented as shown in Table 3 below.

**TABLE 3**

| Linker | | TGT | TTGT |
|---|---|---|---|
| Standard literature for linker determination | | Based on US 2008/0318887 A1(Dec. 25, 2008) | Based on Nucleic Acids Research, 2010, 38(14), 4877-4888 |
| GRO aptamer sequence structure | | [GGT]ₘ-TGT-[GGT]ₙ-GG | [GGT]ₘ-GG-TTGT-[GGT]ₙ-GG |
| Position of modified nucleic acid in oligonucleot ides of the present disclosure | 5'-position | [M]ₘ-[N¹]ₙ₁-[GGT]ₓ-N²N³N⁴-[GGT]_{y}-GG-[N⁵]ₙ₅ | [M]ₘ-[N¹]ₙ₁-[GGT]ₘ-GG-N²N³N⁴N⁵-[GGT]ₙ-GG-[N⁶]ₙ₆ |
| | 3'- position | [N¹]ₙ₁-[GGT]ₓ-N²N³N⁴-[GGT]_{y}-GG-[N⁵]ₙ₅-[M]ₘ | [N¹]ₙ₁-[GGT]ₘ-GG-N²N³N⁴N⁵-[GGT]ₙ-GG-[N⁶]ₙ₆-[M]ₘ |
| | Internal modificati on | In [M]ₘ-[N¹]ₙ₁-[GGT]ₓ-N²N³N⁴-[GGT]_{y}-GG-[N⁵]ₙ₅T (N = T inclusive), substitution with at least one T' (modified nucleic acid) may be made | In [M]ₘ-[N¹]ₙ₁-[GGT]ₘ-GG-N²N³N⁴N⁵-[GGT]ₙ-GG-[N⁶]ₙ₆T (N = T inclusive), substitution with at least one T' (modified nucleic acid) may be made. |
| | Orthogonal modificati on | In [M]ₘ-[N¹]ₙ₁-[GGT]ₓ-N²N³N⁴-[GGT]_{y}-GG-[N⁵]ₙ₅T (N = T inclusive), substitution with at least one (e.g., 5-modified dU) may be made. | In [M]ₘ-[N¹]ₙ₁-[GGT]ₘ-GG-N²N³N⁴N⁵-[GGT]ₙ-GG-[N⁶]ₙ₆T (N = T inclusive), substitution with at least one (e.g., 5-modified dU) may be made |

In an embodiment, in Table 3, N1, N2, N3, N4, N5, and N6 may each be a nucleic acid molecule and may be any one of the naturally occurring unmodified nucleotides A, T, C, G, U, or their modified forms.

In an embodiment, each of the nucleic acid nucleotides comprised in the oligonucleotide sequence may be substituted with a modified nucleic acid or modified nucleotide, and the oligonucleotide sequence may comprise one or more modified nucleic acids or modified nucleotides within its sequence.

In an embodiment, in the Chemical Formula for the oligonucleotide or its derivatives, n, m, x, y, n1, n5, n6 may each be an integer from 1 to 10, from 1 to 9, from 1 to 8, from 1 to 7, from 1 to 6, from 1 to 5, from 1 to 4, from 1 to 3, or from 1 to 2. In an embodiment, n5 or n6 may each be 0.

In an embodiment, for example, the oligonucleotide can be as indicated in Table 4 below. In Table 4, Gem stands for gemcitabine.

**TABLE 4**

| Linker | TGT | TTGT |
|---|---|---|
| Compound 11 (IO101L) | [Gem] [Gem]-T-GGT-GGT-GGT-GGT-**TGT**-GGT-GGT-GGT-GGT-GG | [Gem] [Gem]-T-GGT-GGT-GGT-GG-**TTGT**-GGT-GGT-GGT-GGT-GG |
| Structure of compound 11 | [Gem]₂-T-[GGT]₄-TGT-[GGT]₄-GG | [Gem]₂-T-[GGT]₃-GG-TTGT-[GGT]₄-GG |
| | [M]ₘ-[N¹]ₙ₁-[GGT]ₓ-N²N³N⁴-[GGT]_{y}-GG-[N⁵]ₙ₅M = Gem, m = 2N¹ = T, n¹ = 1, N⁵ = none, n⁵ = 0N² = T, N³ = G, N⁴ = Tx = 4, y = 4 | [M]ₘ-[N¹]ₙ₁-[GGT]ₘ-GG-N²N³N⁴N⁵-[GGT]ₙ-GG-[N⁶]ₙ₆M = Gem, m = 2N¹ = T, n¹ = 1, N⁶ = none, n⁶ = 0, N² = T, N³ = T, N⁴ = G, N⁵ = Tx = 3, y = 4 |

In an embodiment, triple-negative breast cancer may be primary and/or metastatic triple-negative breast cancer. In an embodiment, metastatic triple-negative breast cancer may refer to cancer or tumors that have metastasized from primary triple-negative breast cancer originating in the breast to other tissues or organs of the subject. Such metastatic triple-negative breast cancer can occur even after the primary triple-negative breast cancer has been surgically resected from the affected area of the subject, and such metastasis can act as one of the causes for the poor prognosis of triple-negative breast cancer.

In an embodiment, the pharmaceutical composition may be administered to a subject who has undergone surgical resection of triple-negative breast cancer. In an embodiment, the pharmaceutical composition may be for preventing or treating metastatic cancer or tumors in the subject who has undergone such surgical resection. In an embodiment, the metastatic cancer may be lung cancer, brain cancer, osteosarcoma, liver cancer, or lymphoma, but is not limited thereto.

In an embodiment, the pharmaceutical composition may be implanted at the surgical resection site of triple-negative breast cancer.

In an embodiment, the pharmaceutical composition can be a drug delivery composition.

An embodiment of the present disclosure pertains to a drug delivery device containing the pharmaceutical composition.

An embodiment of the present disclosure pertains to a method for preventing or treating triple-negative breast cancer, the method including the step of administering the modified oligonucleotide or pharmaceutical composition of the present disclosure to a subject. In an embodiment, the subject may be one who has undergone surgical resection of triple-negative breast cancer.

An embodiment of the present disclosure pertains to a use of the modified oligonucleotide or pharmaceutical composition of the present disclosure for preventing or treating triple-negative breast cancer.

An embodiment of the present disclosure pertains to the modified oligonucleotide or pharmaceutical composition of the disclosure for use in a method for preventing or treating triple-negative breast cancer.

An embodiment of the present disclosure relates to a pharmaceutical composition for preventing or treating metastatic cancer or tumors that occur after the surgical resection of triple-negative breast cancer (TNBC), comprising a modified oligonucleotide or a pharmaceutically acceptable salt thereof. An embodiment of the present disclosure pertains to a pharmaceutical composition for inhibiting the metastasis of cancer or tumors after the surgical resection of triple-negative breast cancer (TNBC), comprising a modified oligonucleotide or a pharmaceutically acceptable salt thereof.

An embodiment of the present disclosure relates to a method for preventing or treating metastatic cancer or tumors that occur after the surgical resection of triple-negative breast cancer (TNBC), comprising a step of administering the modified oligonucleotide or pharmaceutical composition of the present disclosure to a subject. An embodiment of the present disclosure relates to a method for inhibiting the metastasis of cancer or tumors after the surgical resection of triple-negative breast cancer (TNBC), comprising a step of administering the modified oligonucleotide or pharmaceutical composition of the present disclosure to a subject.

An embodiment of the present disclosure relates to a use of the modified oligonucleotide or pharmaceutical composition of the present disclosure for preventing or treating metastatic cancer or tumors that occur after the surgical resection of triple-negative breast cancer (TNBC). An embodiment of the presents disclosure relates to a use of the modified oligonucleotide or pharmaceutical composition of the present disclosure for inhibiting the metastasis of cancer or tumors after the surgical resection of triple-negative breast cancer (TNBC).

An embodiment of the present disclosure relates to the modified oligonucleotide or pharmaceutical composition of the present disclosure for use in a method for preventing or treating metastatic cancer or tumors that occur after the surgical resection of triple-negative breast cancer (TNBC). An embodiment of the present disclosure relates to the modified oligonucleotide or pharmaceutical composition of the present disclosure for use in a method for inhibiting the metastasis of cancer or tumors after the surgical resection of triple-negative breast cancer (TNBC).

In an embodiment, the step of administering or delivery may comprise implanting the pharmaceutical composition in the subject, specifically implanting the pharmaceutical composition at the site of surgical resection of triple-negative breast cancer.

An embodiment of the present disclosure relates to a method for preventing the regrowth of primary triple-negative breast cancer by surgically implanting the pharmaceutical composition, drug delivery composition, or drug delivery device of the present disclosure. An embodiment of the present disclosure relates to a method for preventing the recurrence and/or metastasis of triple-negative breast cancer by surgically implanting the pharmaceutical composition, drug delivery composition, or drug delivery device of the present disclosure.

In an embodiment, the method may further comprise implanting the pharmaceutical composition, drug delivery composition, or drug delivery device after the surgical resection of the triple-negative breast cancer tumor. In an embodiment, the method may further comprise implanting the pharmaceutical composition, drug delivery composition, or drug delivery device at the resection site of the triple-negative breast cancer tumor.

The matters described in the pharmaceutical compositions, uses, and treatment methods of the present disclosure can be applied equally, as long as they are not contradictory.

In an embodiment, the pharmaceutical composition, drug delivery composition, or drug delivery device may comprise one or more of a biomaterial, a pharmaceutically acceptable excipient, a pharmaceutically acceptable carrier, and a pharmaceutically acceptable diluent.

In an embodiment, the biomaterial may be a scaffold or a depot. The scaffold or depot may include any synthetic or naturally occurring material suitable for containing and enhancing the sustained or extended release of any therapeutic agents within the pharmaceutical composition, drug delivery composition, or drug delivery device as described herein. In an embodiment, the biomaterial may possess characteristics that provide favorable properties (for example, storage modulus, biodegradability, release profile of therapeutic agents) to the compositions and devices described herein. In an embodiment, the biomaterial can extend the release of specific therapeutics within the solution compared to the delivery of specific therapeutics at the tumor resection site. In an embodiment, biomaterials can extend the release of specific therapeutics at the tumor resection site by at least 5 minutes, 10 minutes, 20 minutes, 30 minutes, 40 minutes, 50 minutes, 60 minutes, 2 hours, 3 hours, 4 hours, 5 hours, 6 hours, 7 hours, 8 hours, 9 hours, 10 hours, 11 hours, 12 hours, 18 hours, 24 hours, 2 days, 3 days, 4 days, 5 days, 6 days, 7 days, 2 weeks, 3 weeks, or 4 weeks.

In an embodiment, examples of the biomaterial include hyaluronic acid, alginate, chitosan, chitin, chondroitin sulfate, dextran, gelatin, hydrogel, collagen, starch, cellulose, polysaccharide, fibrin, ethylene-vinyl acetate (EVA), poly(lactic-co-glycolic acid) (PLGA), polylactic acid (PLA), polyglycolic acid (PGA), polyethylene glycol (PEG), PEG diacrylate (PEGDA), disulfide-containing PEGDA (PEGSSDA), PEG dimethacrylate (PEGDMA), polydioxanone (PDO), polyhydroxybutyrate (PHB), poly(2-hydroxyethyl methacrylate) (pHEMA), polycaprolactone (PCL), poly(beta-amino ester) (PBAE), poly(ester amide), polypropylene glycol) (PPG), poly(aspartic acid), poly(glutamic acid), polypropylene fumarate) (PPF), poly(sebacic anhydride) (PSA), poly(trimethylene carbonate) (PTMC), poly(desaminotyrosyltyrosine alkyl ester carbonate) (PDTE), poly[bis(trifluoroethoxy)phosphazene], polyoxymethylene, single-walled carbon nanotube, polyphosphazene, polyanhydride, poly(N-vinyl-2-pyrrolidone) (PVP), poly(vinyl alcohol) (PVA), poly(acrylic acid) (PAA), poly(methacrylic acid) (PMA), polyacetal, poly(alpha ester), poly(ortho ester), polyphosphoester, polyurethane, polycarbonate, polyamide, polyhydroxyalkanoate, polyglycerol, polyglucuronic acid, derivatives thereof, and/or combinations thereof, but are not limited thereto.

In an embodiment, the biomaterial may be a hydrophobic polymer. In an embodiment, examples of the hydrophobic polymer include ethylene-vinyl acetate (EVA), poly(lactic-co-glycolic acid) (PLGA), polylactic acid (PLA), polyglycolic acid (PGA), polydioxanone (PDO), polyhydroxybutyrate (PHB), polycaprolactone (PCL), poly(ester amide), polypropylene fumarate) (PPF), poly(sebacic anhydride) (PSA), poly(trimethylene carbonate) (PTMC), poly(desaminotyrosyltyrosine alkyl ester carbonate) (PDTE), poly[bis(trifluoroethoxy)phosphazene], polyoxymethylene, single-walled carbon nanotubes, polyphosphazene, poly(anhydride), poly(N-vinyl-2-pyrrolidone) (PVP), poly(acrylic acid) (PAA), poly(methacrylic acid) (PMA), poly(alpha ester), poly(ortho ester), polyphosphoester, polyurethane, polycarbonate, polyamide, or polyhydroxyalkanoate, but are not limited thereto. Using a hydrophobic polymer as a biomaterial may be particularly useful when the therapeutic(s) in the pharmaceutical composition, drug delivery composition, or drug delivery device is hydrophilic. Hydrophobic therapeutics are expected to be released over a longer period (e.g., several days/weeks) compared to the more beneficial release timescale for therapeutic effect (e.g., several hours). Therefore, in an embodiment where the biomaterial is a hydrophobic polymer, the therapeutic(s) of the pharmaceutical composition, drug delivery composition, or drug delivery device can be hydrophilic molecules.

The term "biomaterial," as used herein, refers to any biocompatible material manipulated to interact with biological systems for a medical purpose, such as therapeutic or diagnostic purposes. Here, biomaterials can be derived from nature or synthesized.

As used herein, the term "hydrogel" refers to a network structure of polymer chains that are hydrophilic, sometimes found as a colloidal gel in which water is the dispersion medium. Hydrogels are highly absorbent (they can contain over 90% water) natural or synthetic polymeric network structure. Hydrogels also possess a degree of flexibility similar to natural tissue due to their significant water content.

The terms "implantable," "implantation," "implanting," and "implant,", as used herein, refer to positioning a pharmaceutical composition, drug delivery composition, or drug delivery device at a specific location in a subject, such as within a tumor resection site or in a sentinel lymph node, and typically by general surgical methods.

The term "biocompatible,", as used herein, refers to a material that is substantially non-toxic in the in vivo environment of its intended use and that is not substantially rejected by the patient's physiological system (i.e., is non-antigenic). This can be gauged by the ability of a specific material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity, and/or immunogenicity. A biocompatible structure or material, when introduced into a majority of patients, will not cause an undesirably adverse, long-lived, or escalating biological reaction or response and is distinguished from a mild, transient inflammation, which typically accompanies surgery or implantation of foreign objects into a living organism.

In an embodiment, the pharmaceutical composition may comprise a collagen dispersion. In an embodiment, the collagen dispersion may contain from 0.5 to 5.5 g, from 1 to 4.5 g, or from 2 to 3.5 g of collagen per 100 ml of PBS solution. The concentration of collagen in the collagen dispersion may be from 0.6 to 6.0%, from 0.5 to 5.5%, from 0.5 to 3.0%, from 1 to 4.5%, or from 2 to 3.5%. In an embodiment, the concentration of collagen comprised in the pharmaceutical composition may affect the duration of the drug's effect or its therapeutic efficacy, so it is important that the collagen is comprised at an appropriate concentration.

In an embodiment, the collagen comprised in the pharmaceutical composition of the present disclosure may exist in a form that envelops the modified oligonucleotide comprised in the composition.

In an embodiment, the pharmaceutical composition of the present disclosure may be manufactured or used in various formulations. In an embodiment, these formulations may be powder, granule, tablet, emulsion, syrup, aerosol, soft or hard gelatin capsule, sterile injection solution, sterile powder, sol-gel formulation, scaffold form, or patch form (disk form), but with no limitations thereto. In an embodiment, the pharmaceutical composition of the present disclosure may be in the form of a collagen dispersion or a hydrogel dispersion. In an embodiment, the pharmaceutical composition of the present disclosure may be in the form of a sol-gel, scaffold form, patch form, or disk form. In an embodiment, the pharmaceutical composition of the present disclosure may be administered in the form of the formulations.

In an embodiment, the pharmaceutical composition may be a non-oral composition. Non-oral compositions may be advantageously formulated in a unit dosage form for uniformity of dosage and ease of administration.

In an embodiment, the pharmaceutical composition of the present disclosure may be administered to an individual through any route including, but not limited to, intravenous (IV), intraperitoneal (IP), intraocular, intranasal, intra-arterial, intra-pulmonary, oral, inhalation, intravesical, vaginal, intradermal, intramuscular (IM), endotracheal, subcutaneous (SC), intraspinal, transdermal, transpleural, topical, mucosal (e.g., via nasal mucosa), gastrointestinal, intra-articular, intra-cystic, intraventricular, intracranial, intraurethral, intrahepatic, and intratumoral (IT). In an embodiment, the pharmaceutical composition of the present disclosure may be surgically implanted into the subject. In an embodiment, the pharmaceutical composition of the present disclosure may be surgically implanted into the subject at the site or area where the tumor was resected. In an embodiment, the composition of the present disclosure may be administered through all common routes, such as infusion or bolus injection, absorption through epithelial or mucocutaneous linings (e.g., oral mucosa, rectal mucosa, and intestinal mucosa), and may also be administered with other biologically active ingredients. The administration may be systemic or local.

In an embodiment, the content of the modified oligonucleotide or its pharmaceutically acceptable salt comprised in the pharmaceutical composition, drug delivery composition, or drug delivery device for the prevention or treatment of triple-negative breast cancer is a content useful for prevention or treatment of cancer and can be appropriately adjusted according to the condition and/or severity of the disease of the subject. In an embodiment, the optimal effective dose of the modified oligonucleotide or its pharmaceutically acceptable salt, or the pharmaceutical composition containing them, can be determined experimentally and may vary depending on the type and severity of the disease, route of administration, disease progression and health, and individual's body weight and surface area. This determination is within the skill of the ordinary artisan.

In an embodiment, the modified oligonucleotide or its pharmaceutically acceptable salt may be administered, for example, at a dose of about 0.05 ng to about 100 mg per kg of body weight per day. In an embodiment, the dosage of the modified oligonucleotide or its pharmaceutically acceptable salt may be about 0.01 mg/kg or more, about 0.1 mg/kg or more, about 0.5 mg/kg or more, about 1.0 mg/kg or more, about 1.5 mg/kg or more, about 2.0 mg/kg or more, about 2.5 mg/kg or more, about 3.0 mg/kg or more, about 3.5 mg/kg or more, about 4.0 mg/kg or more, about 4.5 mg/kg or more, about 5.0 mg/kg or more, about 6.0 mg/kg or more, about 8.0 mg/kg or more, about 10.0 mg/kg or more, about 15.0 mg/kg or more, or about 20.0 mg/kg or less, about 17.0 mg/kg or less, about 14.0 mg/kg or less, about 11.0 mg/kg or less, about 9.0 mg/kg or less, about 7.0 mg/kg or less, about 5.5 mg/kg or less, about 5.0 mg/kg or less, about 4.5 mg/kg or less, about 4.0 mg/kg or less, about 3.5 mg/kg or less, about 3.0 mg/kg or less, about 2.5 mg/kg or less, about 2.0 mg/kg or less, about 1.5 mg/kg or less, or about 1.0 mg/kg or less. In an embodiment, the dosage of the modified oligonucleotide or its pharmaceutically acceptable salt may be about 1.0 mg/kg to about 10.0 mg/kg or about 1.0 mg/kg to about 5.0 mg/kg.

In an embodiment, the effective dose of the modified oligonucleotide of the present disclosure may be about 0.01 mg to about 1,000 mg. In an embodiment, the pharmaceutical composition of the present disclosure may contain the modified oligonucleotide of the present disclosure in a single dose of about 0.001 mg, about 0.01 mg, about 0.05 mg, about 0.1 mg, about 0.5 mg, about 1 mg, about 5 mg, about 10 mg, about 15 mg, about 30 mg, about 50 mg, about 100 mg, about 200 mg, about 300 mg, about 400 mg, about 500 mg, about 600 mg, about 700 mg, about 800 mg, about 900 mg, or about 1,000 mg (inclusive, including any value between these numbers).

In an embodiment, the modified oligonucleotide or pharmaceutical composition containing same according to the present disclosure may be administered as a single daily dose or the total daily dose may be administered in divided doses 2, 3, or 4 times a day. In an embodiment, the pharmaceutical composition of the present disclosure can be administered 6 times a week, 5 times a week, 4 times a week, 3 times a week, 2 times a week, once a week, once every other week, once every 3 weeks, once a month, once every 2 months, once every 3 months, once every 6 months, once every 9 months, or once a year. In an embodiment, the pharmaceutical composition of the present disclosure can be administered less frequently, for example, once every 3 months, 4 months, 5 months, 6 months, 7 months, 8 months, 9 months, 10 months, 11 months, or once a year.

In an embodiment, the modified oligonucleotide or pharmaceutical composition containing it of the present disclosure can be used alone or in combination with one or more additional therapeutic agents, and in such cases, it can be administered singly or multiply. For example, the modified oligonucleotide or pharmaceutical composition containing same of the present disclosure can be administered alone or in combination with other therapeutic agents that demonstrate anticancer activity. In an embodiment, the combination can be provided as a simultaneous administration, where the modified oligonucleotide and at least one therapeutic agent are administered together in the same composition or simultaneously in different compositions. In an embodiment, the combination can be provided as a separate administration, where the administration of the modified oligonucleotide or pharmaceutical composition containing it can occur before, during, or after the administration of at least one therapeutic agent. The interval between sequential administrations can be at least a few minutes, a few hours, or a few days (or alternatively, less). In an embodiment, the pharmaceutical composition of the present disclosure can be used in combination with other treatment modalities.

An embodiment of the present disclosure may relate to a method for manufacturing the pharmaceutical composition for treating triple-negative breast cancer of the present disclosure.

In an embodiment, the method of manufacturing the pharmaceutical composition comprises the step of preparing a dispersion containing the modified oligonucleotide. In an embodiment, the dispersion containing the modified oligonucleotide can be manufactured by mixing the modified oligonucleotide with PBS. In an embodiment, the method of manufacturing the pharmaceutical composition can further comprise the step of mixing the dispersion containing the modified oligonucleotide with a collagen dispersion or hydrogel dispersion. In an embodiment, the dispersion containing the modified oligonucleotide can be manufactured by mixing the modified oligonucleotide with PBS.

In an embodiment, the aforementioned collagen content can be similarly applicable to biomaterials.

An embodiment of the present disclosure may relate to a manufactured item or kit containing the modified oligonucleotide or pharmaceutical composition containing it of the present disclosure within suitable packaging. In an embodiment, the suitable packaging is well known in the field and may include, for example, vials (such as sealed vials), containers, ampoules, bottles, jars, flexible packaging (for example, sealed Mylar or plastic bags), etc., but is not limited to these. Such manufactured items may additionally be sterilized and/or sealed.

In an embodiment, the kit may further comprise instructions for using the composition for the methods or uses described in the present disclosure. In an embodiment, the kit may further comprise other components that are desirable from a commercial and user perspective, for example, other buffers, diluents, filters, needles, syringes, and package inserts (PI) containing instructions for performing any methods described in the present disclosure. For example, in an embodiment, the kit may comprise (i) the modified oligonucleotide described in the present disclosure, (ii) a pharmaceutically acceptable carrier, and (iii) one or more of the following: buffers, diluents, filters, needles, syringes, and a package insert containing instructions for administration.

A better understanding of the present disclosure may be obtained through the following examples that are set forth to illustrate, but are not to be construed to limit, the present disclosure.

### [EXAMPLE 1] Construction of Modified Oligonucleotide

### Example 1-1. Synthesis of oligonucleotide conjugated with gemcitabine

Twenty-eight oligonucleotide species were designed and manufactured, and gemcitabine was bound to each of the manufactured oligonucleotides to produce modified oligonucleotides. The sequences of the 28 designed oligonucleotides and the 28 species of modified oligonucleotides with modified nucleic acids bound are shown in Table 6 below. Hereafter, gemcitabine may also be expressed as Gem.

**TABLE 6**

| | Oligonucleotide (5'→ 3') | Modified Oligonucleotide (5'→ 3') |
|---|---|---|
| 1 | [TGG]₁[TTG][TGG]₁ (SEQ ID NO: 1) | (Gem)₂-[TGG]₁[TTG][TGG]₁ (Compound 1) |
| 2 | [TGG]₁[TTG][TGG]₂ (SEQ ID NO: 2) | (Gem)₂-[TGG]₁[TTG][TGG]₂ (Compound 2) |
| 3 | [TGG]₂[TTG][TGG]₁ (SEQ ID NO: 3) | (Gem)₂-[TGG]₂[TTG][TGG]₁ (Compound 3) |
| 4 | [TGG]₂[TTG][TGG]₂ (SEQ ID NO: 4) | (Gem)₂-[TGG]₂[TTG][TGG]₂ (Compound 4) |
| 5 | [TGG]₂[TTG][TGG]₃ (SEQ ID NO: 5) | (Gem)₂-[TGG]₂[TTG][TGG]₃ (Compound 5) |
| 6 | [TGG]₃[TTG][TGG]₂ (SEQ ID NO: 6) | (Gem)₂-[TGG]₃[TTG][TGG]₂ (Compound 6) |
| 7 | [TGG]₃[TTG][TGG]₃ (SEQ ID NO: 7) | (Gem)₂-[TGG]₃[TTG][TGG]₃ (Compound 7) |
| 8 | [TGG]₃[TTG][TGG]₄ (SEQ ID NO: 8) | (Gem)₂-[TGG]₃[TTG][TGG]₄ (Compound 8) |
| 9 | [TGG]₄[TTG][TGG]₃ (SEQ ID NO: 9) | (Gem)₂-[TGG]₄[TTG][TGG]₃ (Compound 9) |
| 10 | TGG₄TTGTGG₄ (SEQ ID NO: 10) | (Gem)₂-[TGG]₄[TTG][TGG]₄ (Compound 10) |
| 11 | [TGG]₄[TTG][TGG]₅ (SEQ ID NO: 11) | (Gem)₂-[TGG]₄[TTG][TGG]₅ (Compound 11) |
| 12 | [TGG]₅[TTG][TGG]₄ (SEQ ID NO: 12) | (Gem)₂-[TGG]₅[TTG][TGG]₄ (Compound 12) |
| 13 | [TGG]₅[TTG][TGG]₅ (SEQ ID NO: 13) | (Gem)₂-[TGG]₅[TTG][TGG]₅ (Compound 13) |
| 14 | [TGG]₅[TTG][TGG]₆ (SEQ ID NO: 14) | (Gem)₂-[TGG]₅[TTG][TGG]₆ (Compound 14) |
| 15 | [TGG]₆[TTG][TGG]₅ (SEQ ID NO: 15) | (Gem)₂-[TGG]₆[TTG][TGG]₅ (Compound 15) |
| 16 | TGG₆TTGTGG₆ (SEQ ID NO: 16) | (Gem)₂-[TGG]₆[TTG][TGG]₆ (Compound 16) |
| 17 | [TGG]₆[TTG][TGG]₇ (SEQ ID NO: 17) | (Gem)₂-[TGG]₆[TTG][TGG]₇ (Compound 17) |
| 18 | [TGG]₇[TTG][TGG]₆ (SEQ ID NO: 18) | (Gem)₂-[TGG]₇[TTG][TGG]₆ (Compound 18) |
| 19 | [TGG]₇[TTG][TGG]₇ (SEQ ID NO: 19) | (Gem)₂-[TGG]₇[TTG][TGG]₇ (Compound 19) |
| 20 | [TGG]₇[TTG][TGG]₈ (SEQ ID NO: 20) | (Gem)₂-[TGG]₇[TTG][TGG]₈ (Compound 20) |
| 21 | [TGG]₈[TTG][TGG]₇ (SEQ ID NO: 21) | (Gem)₂-[TGG]₈[TTG][TGG]₇ (Compound 21) |
| 22 | [TGG]₈[TTG][TGG]₈ (SEQ ID NO: 22) | (Gem)₂-[TGG]₈[TTG][TGG]₈ (Compound 22) |
| 23 | [TGG]₈[TTG][TGG]₉ (SEQ ID NO: 23) | (Gem)₂-[TGG]₈[TTG][TGG]₉ (Compound 23) |
| 24 | [TGG]₉[TTG][TGG]₈ (SEQ ID NO: 24) | (Gem)₂-[TGG]₉[TTG][TGG]₈ (Compound 24) |
| 25 | [TGG]₉[TTG][TGG]₉ (SEQ ID NO: 25) | (Gem)₂-[TGG]₉[TTG][TGG]₉ (Compound 25) |
| 26 | [TGG]₉[TTG][TGG]₁₀ (SEQ ID NO: 26) | (Gem)₂-[TGG]₉[TTG][TGG]₁₀ (Compound 26) |
| 27 | [TGG]₁₀[TTG][TGG]₉ (SEQ ID NO: 27) | (Gem)₂-[TGG]₁₀(TTG][TGG]₉ (Compound 27) |
| 28 | [TGG]₁₀[TTG][TGG]₁₀ (SEQ ID NO: 28) | (Gem)₂-[TGG]₁₀[TTG][TGG]₁₀(Compound 28) |

The 'oligonucleotides bearing gemcitabine' (modified oligonucleotides) were synthesized using solid-phase phosphoramidite chemistry with a MerMade 12 DNA synthesizer (BioAutomation Manufacturing, Irving, TX).

Desalting was performed using Biotage MPLC and a C18 cartridge. The compounds were dissolved in distilled water (D.W.) and separated/purified using Waters Acquity UPLC H-Class with Xbridge Oligonucleotide BEH C18 130A, 1.7 µm, 2.1x50 mm column, column oven temperature of 50oC, mobile phase A solvent (0.1M TEAA), B solvent (100% ACN), and flow of 0.3 mL/min. The molecular weight was identified using a Waters G2-XS Q-TOF mass spectrometer. All oligonucleotide syntheses were performed in-house.

### Example 1-2. Synthesis of different types of modified oligonucleotides

Using the same method described in 1-1 above, ((N)ₓ-[TGG]₄[TTG][TGG]_{4/or5}, ([TGG]₄[TTG][TGG]_{4/or5}-(N)ₓ), and ((N)ₓ-[TGG]₄[TTG][TGG]_{4/or5}-(N)_{y}) were synthesized (see Table 7 below).

**TABLE 7**

| Modified nucleic acid (N) type | Modified oligonucleotide | Obs.MW(g/mol) | ExactMW(g/mol) |
|---|---|---|---|
| 2¹,2¹-difluorodeoxycytidine | (N)₁-[TGG]₄[TTG][TGG]₄ | 8906.63 | 8897.4251 |
| | (N)₂-[TGG]₄[TTG][TGG]₄ | 9221.80 | 9222.4526 |
| | (N)₃-[TGG]₄[TTG][TGG]₄ | 9556.97 | 9547.4801 |
| | (N)₄-[TGG]₄[TTG][TGG]₄ | 9871.13 | 9872.5077 |
| | (N)₅-[TGG]₄[TTG][TGG]₄ | 10206.30 | 10197.5352 |
| | (N)₁-[TGG]₄[TTG][TGG]₅ | 9858.24 | 9859.5762 |
| | (N)₂-[TGG]₄[TTG][TGG]₅ | 10183.41 | 10184.6037 |
| | (N)₃-[TGG]₄[TTG][TGG]₅ | 10509.57 | 10509.6312 |
| | (N)₄-[TGG]₄[TTG][TGG]₅ | 10833.74 | 10834.6587 |
| | (N)₅-[TGG]₄[TTG][TGG]₅ | 11158.90 | 11159.6863 |
| 5-fluoro-deoxyuridine | (N)₁-[TGG]₄[TTG][TGG]₄ | 8879.63 | 8880.4185 |
| | (N)₅-[TGG]₄[TTG][TGG]₄ | 10111.26 | 10112.5024 |
| | (N)₁₀-[TGG]₄[TTG][TGG]₄ | 11651.05 | 11652.6072 |
| | (N)₁-[TGG]₄[TTG][TGG]₅ | 9841.23 | 9842.5696 |
| | (N)₅-[TGG]₄[TTG][TGG]₅ | 11073.87 | 11074.6535 |
| | (N)₁₀-[TGG]₄[TTG][TGG]₅ | 12613.66 | 12614.7583 |
| cytosine arabinoside | (N)₁-[TGG]₄[TTG][TGG]₄ | 8877.66 | 8878.4466 |
| | (N)₂-[TGG]₄[TTG][TGG]₄ | 9183.85 | 9184.4958 |
| | (N)₃-[TGG]₄[TTG][TGG]₄ | 9490.04 | 9490.5449 |
| | (N)₁-[TGG]₄[TTG][TGG]₅ | 9839.26 | 9840.5977 |
| | (N)₂-[TGG]₄[TTG][TGG]₅ | 10145.45 | 10146.6469 |
| | (N)₃-[TGG]₄[TTG][TGG]₅ | 10451.64 | 10452.6960 |

| Modified nucleic acid (N) type | Modified oligonucleotide ([TGG]ₘ[TTG][TGG]ₙ₋(N)ₓ) | | |
|---|---|---|---|
| 2',2'-difluorodeoxycytidine | [TGG]₄[TTG][TGG]₄-(N)₁ | 8906.63 | 8897.4251 |
| | [TGG]₄[TTG][TGG]₄-(N)₂ | 9221.80 | 9222.4526 |
| | [TGG]₄[TTG][TGG]₄-(N)₃ | 9546.97 | 9547.4801 |
| | [TGG]₄[TTG][TGG]₄-(N)₄ | 9871.13 | 9872.5077 |
| | [TGG]₄[TTG][TGG]₄-(N)₅ | 10196.30 | 10197.5352 |
| | [TGG]₄[TTG][TGG]₅-(N)₁ | 9858.24 | 9859.5762 |
| | [TGG]₄[TTG][TGG]₅-(N)₂ | 10183.41 | 10184.6037 |
| | [TGG]₄[TTG][TGG]₅-(N)₃ | 10508.57 | 10509.6312 |
| | [TGG]₄[TTG][TGG]₅-(N)₄ | 10833.74 | 10834.6587 |
| | [TGG]₄[TTG][TGG]₅-(N)₅ | 11158.90 | 11159.6863 |
| 5-fluoro-deoxyuridine | [TGG]₄[TTG][TGG]₄-(N)₁ | 8880.63 | 8880.4185 |
| | [TGG]₄[TTG][TGG]₄-(N)₅ | 10111.26 | 10112.5024 |
| | [TGG]₄[TTG][TGG]₄-(N)₁₀ | 11651.05 | 11652.6072 |
| | [TGG]₄[TTG][TGG]₅-(N)₁ | 9841.23 | 9842.5696 |
| | [TGG]₄[TTG][TGG]₅-(N)₅ | 11073.87 | 11074.6535 |
| | [TGG]₄[TTG][TGG]₅-(N)₁₀ | 12613.66 | 12614.7583 |
| cytosine arabinoside | [TGG]₄[TTG][TGG]₄-(N)₁ | 8877.66 | 8878.4466 |
| | [TGG]₄[TTG][TGG]₄-(N)₂ | 9183.85 | 9184.4958 |
| | [TGG]₄[TTG][TGG]₄-(N)₃ | 9490.04 | 9490.5449 |
| | [TGG]₄[TTG][TGG]₅-(N)₁ | 9839.26 | 9840.5977 |
| | [TGG]₄[TTG][TGG]₅-(N)₂ | 10145.45 | 10146.6469 |
| | [TGG]₄[TTG][TGG]₅-(N)₃ | 10451.64 | 10452.6960 |

| Modified nucleic acid (N) type | Modified oligonucleotide ((N)ₓ-[TGG]ₘ[TTG][TGG]ₙ-(N)x) | | |
|---|---|---|---|
| 2',2'-difluorodeoxycytidine | (N)₁-[TGG]₄[TTG][TGG]₄-(N)₁ | 9221.80 | 9222.4526 |
| | (N)₃-[TGG]₄[TTG][TGG]₄-(N)₃ | 10521.46 | 10522.5627 |
| | (N)₅-[TGG]₄[TTG][TGG]₄-(N)₅ | 11821.13 | 11822.6728 |
| | (N)₁-[TGG]₄[TTG][TGG]₅-(N)₁ | 10183.41 | 10184.6037 |
| | (N)₃-[TGG]₄[TTG][TGG]₅-(N)₃ | 11483.07 | 11484.7138 |
| | (N)₅-[TGG]₄[TTG][TGG]₅-(N)₅ | 12783.73 | 12784.8239 |
| 5-fluoro-deoxyuridine | (N)₁-[TGG]₄[TTG][TGG]₄-(N)₁ | 9187.79 | 9188.4395 |
| | (N)₃-[TGG]₄[TTG][TGG]₄-(N)₃ | 10419.42 | 10420.5233 |
| | (N)₅-[TGG]₄[TTG][TGG]₄-(N)₅ | 11651.05 | 11652.6072 |
| | (N)₁-[TGG]₄[TTG][TGG]₅-(N)₁ | 10149.39 | 10150.5906 |
| | (N)₃-[TGG]₄[TTG][TGG]₅-(N)₃ | 11381.03 | 11382.6744 |
| | (N)₅-[TGG]₄[TTG][TGG]₅-(N)₅ | 12613.66 | 12614.7583 |
| cytosine arabinoside | (N)₁-[TGG]₄[TTG][TGG]₄-(N)₁ | 9183.85 | 9184.4958 |
| | (N)₃-[TGG]₄[TTG][TGG]₄-(N)₃ | 10407.60 | 10408.6922 |
| | (N)₅-[TGG]₄[TTG][TGG]₄-(N)₅ | 11631.36 | 11632.8887 |
| | (N)₁-[TGG]₄[TTG][TGG]₅-(N)₁ | 10145.45 | 10146.6469 |
| | (N)₃-[TGG]₄[TTG][TGG]₅-(N)₃ | 11369.21 | 11370.8433 |
| | (N)₅-[TGG]₄[TTG][TGG]₅-(N)₅ | 12694.97 | 12595.0398 |

### [EXAMPLE 2] Synthesis of Modified Oligonucleotide (IO101L)

For use in experiments, the modified oligonucleotide (IO101L), which is modified from Compound 11 and has the structure of [Gem]₂-T-[GGT]₄-TGT-[GGT]₄-GG or [Gem]₂-T-[GGT]3-GG-TTGT-[GGT]₄-GG, were prepared.

Specifically, IO101L was synthesized using a standard DNA automatic synthesis method with MerMade 12 (BioAutomation, USA). It was sequentially synthesized by adding one mer (one nucleotide) per cycle of four stages [Deblocking -> Coupling -> Capping -> Oxidation] in the direction from the 3' end to the 5' end. The 5' end of IO101L consisted of two molecules of gemcitabine, which were synthesized using gemcitabine3'-CE phosphoramidite (5'-O-DMT-N4-Bz-2',2'-difluoro-2'-dC-3'-CE Phosphoramidite).

The synthesized nucleic acid ligand was purified using a Prep150 purifier (Waters, USA). For purification a reverse-phase C18 column was used. The purified fraction was analyzed using Waters ACQUITY UPLC H-Class Bio PLUS System (Waters, USA) with a reverse-phase C18 column to collect fractions of purity over 80% and dried them.

Analysis: The final product was analyzed using Waters ACQUITY UPLC H-Class Bio PLUS System to confirm purity, and mass analysis was performed. The mass analysis equipment used was Waters Xevo G2-XS Q-TOF System (Waters, USA).

The effectiveness of IO101L and IO101L-control group against triple-negative breast cancer was evaluated below. The sequences of each substance and the structure of the substance bound with Cy5 fluorophore are as follows. For the IO101L-control group, the nucleic acid sequence of IO101L was altered by substituting all G with C and removing the last TGG unit. Such IO101L-control substances correspond to C-rich oligonucleotides (CRO) with substituted G to C and do not form stable G-quadruplex structures, unlike stable IO101L, hence are structurally unstable and do not exhibit anticancer activity.
IO101L(SEQ ID NO: 29) : (Gem)(Gem)[TGG]₄[TTG][TGG]₅
IO101L-control (SEQ ID NO: 30): (Gem)(Gem)[TCC]₄[TTC][TCC]₄
Cy5-labeled IO101L : Cy5-(Gem)(Gem)[TGG]₄[TTG][TGG]₅
Cy5- labeled IO101L-control: Cy5-(Gem)(Gem)[TCC]₄[TTC][TCC]₅
[Example 3] *In vitro* Efficacy Evaluation of IO101L

### 3-1. Evaluation of Cell Proliferation Inhibition Efficacy of IO101L on Triple Negative Breast Cancer Cell Lines

### 3-1-1. Culture of TNBC Cell Lines and Measurement of Cell Proliferation Inhibition After Drug Treatment

The TNBC cell lines 4T1 (ATCC, USA) and MDA-MB-231 (Korean Cell Line Bank, Korea) were cultured in a 5% CO2, 37°C incubator, and the culture medium was ATCC modified RPMI-1640 (Thermo Scientific, USA) with 10% FBS and 1% antibiotics. At a contingence of 80% or more, the cells were washed with PBS and reacted with 0.05% trypsin-EDTA for 3 minutes. After the reaction, fresh medium was added to inactivate trypsin-EDTA and centrifuged to recover the cells. The recovered cells were subcultured in new medium at a 1:4 ratio. All cells were periodically checked for mycoplasma contamination and only uncontaminated cells were used in experiments.

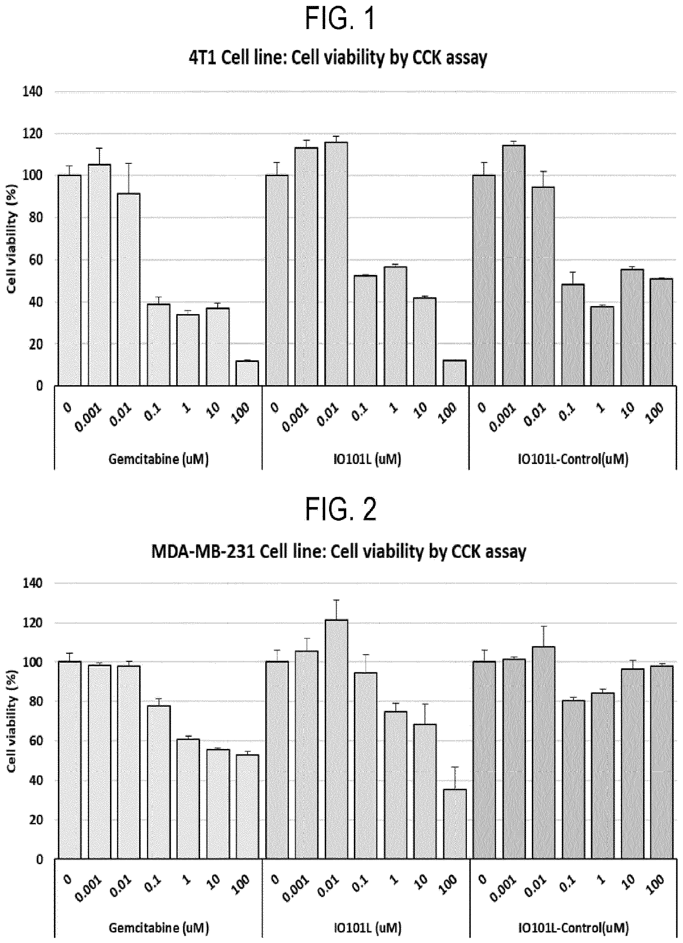

The TNBC cell lines were seeded at a density of 1 × 10⁴ cells/well into a 96-well plate and cultured for 16 hours to allow cell attachment. The medium was replaced with RPMI medium containing 5% FBS and treated with drugs for an additional 72 hours, then 10 µl of WST reagent (Dongin LS, Korea) was added to each well and cultured for 3 hours. Finally, the formed formazan was measured absorbance at 450nm absorbance using a Glomax plate reader (Promega, USA). These results are shown in FIGS. 1 and 2.

### 3-1-2. Measurement of Inhibition Effect

The inhibitory effects of the drugs on 4T1 and MDA-MB-231 cell lines are depicted in FIGS. 1 and 2, respectively. From these results, it was confirmed that the modified oligonucleotides of the present disclosure can effectively inhibit the proliferation of TNBC cells.

### 3-2. Evaluation of Cell Proliferation Inhibition Efficacy of IO101L on HER2 Negative Breast Cancer Cell Lines

### 3-2-1. Culture of HER2 Negative Breast Cancer Cell Lines and Efficacy Evaluation After Drug Treatment

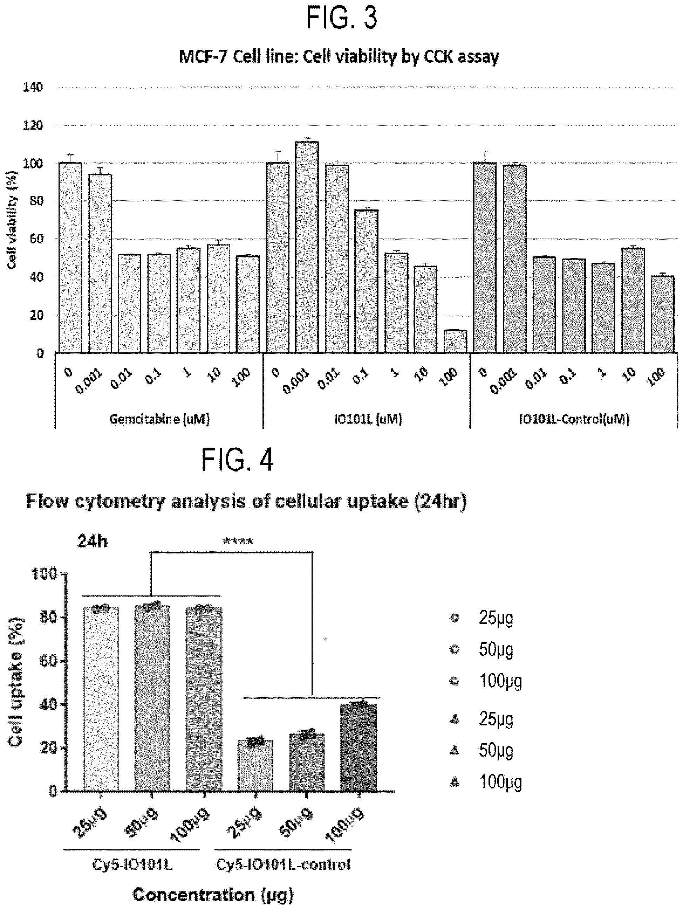

In this experiment, MCF-7 cell line (Korean Cell Line Bank, Korea), which is HER2 negative breast cancer cell line, was used. The cells were cultured and drug-treated, followed by evaluation of cell proliferation inhibition efficacy using the same method described in 3-1-1. These results are shown in FIG. 3.

The drug inhibition effect on the MCF-7 cell line is depicted in FIG. 3. From these results, it was confirmed that the modified oligonucleotides of the present disclosure can effectively inhibit the proliferation of HER2 negative breast cancer cells.

### 3-3. Experiment to Confirm the Intracellular Uptake of IO101L

### 3-3-1. Culture of 4T1 Cell Line and Evaluation of Cell Uptake After Drug Treatment

In this experiment, 4T1 Luc2 cell line (ATCC, USA) was used and the cells were cultured using the same method described in 3-1-1.

The 4T1 Luc2 cell line was seeded at a density of 2 X 10⁵ cells/well in a 24-well plate, and cultured for 16 hours to allow cell attachment. The medium was replaced with RPMI medium containing 5% FBS and treated with drugs for additional 2, 4, 6, 24 hours, and then DAPI staining was performed after the reaction was completed. The finished samples were compared using a flow cytometer (Beckman Coulter CytoFlex (3 lasers)) to measure the degree of fluorescence expression against the control cell line. The results of cell uptake are shown in FIG. 4.

### 3-3-2. Experiment for Cell Uptake of Drug

From the flow cytometry analysis of 4T1 cells, over 80% of the Cy5-labeled IO101L was found to enter the cells after 24 hours. The intracellular influx of the IO101L-treated group was significantly superior to that of the IO101L-control group. From these results, it was confirmed that the modified oligonucleotide of the present disclosure can effectively target TNBC cells and effectively enter TNBC cells.

### [EXAMPLE 4] Preparation of Gemcitabine-Loaded Collagen Patch and IO101L-Loaded Collagen Patch

A collagen drug delivery device in the form of a patch containing the drug (gemcitabine or IO101L) was manufactured by mixing high-purity collagen (COLTRIX^{®} Tendoregen, YuBioSys) dispersion (0.5% to 3.0%) with the drug, pouring same into a circular silicone mold 1 cm in diameter, and freeze-drying. Each collagen patch drug delivery device was manufactured to contain 0.12 mg of gemcitabine, and 0.5mg, 2.0mg, and 4.0mg of IO101L respectively.

Specifically, the drug (e.g., gemcitabine or IO101L) was added to high-purity collagen and then uniformly mixed at room temperature for 30 minutes using a multi-mixer (SLRM-3, MYLAB). The collagen-drug mixture was then aliquoted into cylindrical silicone molds of 1cm diameter. The collagen-drug mixture-filled cylindrical silicone molds were first frozen at -20°C for at least 4 hours. The mixture, once the first freeze was complete, was transferred from the cylindrical silicone molds to sterile dishes or plates and then secondarily frozen for at least 2 hours at -80°C. After pre-freezing until the cold trap temperature of the freeze dryer reached - 80 °C, the mixture was put into the freeze dryer and freeze-dried for at least 16 hours. The freeze-dried mixture was then pressed into a patch shape using acrylic plates, placed into an aluminum pouch, and sealed. The packaged collagen drug delivery device patches were stored refrigerated at 4°C.

Hereafter, the porous material manufactured by the aforementioned method is referred to as a disk or patch.

### [EXAMPLE 5] Evaluation of Anticancer Efficacy of Modified Oligonucleotide/Collagen Composition in Triple-Negative Breast Cancer Orthotopic Animal Model

In evaluating the anticancer efficacy against triple-negative breast cancer, it is crucial to select and use an appropriate *in vivo* experimental model. As immunological and physiological actions in humans are complex, a model similar to the environment in the human body is necessary to rely on non-clinical animal experimental results for anticancer efficacy in humans. This is especially important in the case of triple-negative breast cancer, which is invasive, metastatic, and recurrent, with high probabilities of early distant organ recurrence and mortality. Moreover, to evaluate the anticancer efficacy against metastatic triple-negative breast cancer and cancer metastasis inhibition or recurrence prevention after surgical resection of triple-negative breast cancer, a reliable implementation of cancer metastasis mechanisms is required, making the establishment of the correct non-clinical animal model crucial.

Currently available preclinical spontaneously metastatic TNBC orthotopic murine models include the immunodeficient human MDA-MB-231 model and the immunocompetent mouse 4T1 model. Here, the MDA-MB-231 cell line, derived from human cancer cells, amounts to xenograft transplantation for mice and is thus limited to NOD/SCID (Non-Obese Diabetic-Severe Combined Immunodeficiency) or nude mice. In this regard, xenografted cancer cells are not as invasive or aggressive as experiments done in mice because the host is not human. Typically, Matrigel is used when performing xenografts because human-derived cancer cells do not adhere well and survive in mouse bodies. Moreover, the MDA-MB-231 model, which is a xenografted model constructed by implanting human cells into mice, does not grow as rapidly in mice as mouse cells like 4T1, CT26, MC38, or B16F10. Therefore, invasiveness/aggressiveness of cancer cell is relatively low, leading to less metastasis in mouse models. Also, the use of NOD/SCID or nude mice, which lack some or all immune cells and have the immune system deleted or abnormally operating, limits the reliability of results in the MDA-MB-231 model as a representation of anticancer efficacy in humans. Especially in invasive and metastatic cancers like triple-negative breast cancer, the model may not adequately implement and evaluate cancer occurrence and anticancer mechanisms. Also, as immune cells contribute to the mechanism of cancer metastasis, the MDA-MB-231 model, which necessitates the use of immunodeficient mice, has limitations in evaluating the efficacy against TNBC compared to the 4T1 model and may struggle to yield accurate evaluation results.

Therefore, this experiment aimed to evaluate the preventive or therapeutic effect of the modified oligonucleotide of the present disclosure against triple-negative breast cancer using the 4T1 model. Moreover, the 4T1 model is a triple-negative breast cancer model where 4T1 tumor cells are injected into the 4th fat pad of mice, and after the primary tumor grown is surgically removed completely, the remaining tumor cells spontaneously metastasize to other organs, particularly the lungs, creating a spontaneous metastasis model of breast cancer. When 4T1 cells tagged with the luciferase reporter gene are used, the injected tumor cells can be imaged at various time points using luciferin and an in vivo imaging system (IVIS), making it a useful model for observing the therapeutic effect of the drug against triple-negative breast cancer.

### 5-1. Cell Culture

4T1-Luc2 triple-negative breast cancer cells were obtained from ATCC and cultured in a medium containing 10% FBS and 1% antibiotics at 37°C and 5% CO2. Subculturing was performed when the confluence reached 80% or more. For this, cells were washed with PBS, treated with 0.05% trypsin-EDTA for 3 minutes, and then trypsin was inactivated by adding fresh medium. Cells were harvested by centrifugation and then diluted in new culture vessels at a 1:5 ratio for further culturing. All cells were periodically tested for mycoplasma contamination using PCR methods, and only those confirmed to be free of contamination were used for experiments.

### 5-2. Animal Preparation

Female Balb/c mice (7 weeks old) were purchased from ORIENT BIO (Seongnam, Korea), and all animal experiments were conducted in accordance with the regulations of the Institutional Animal Care and Use Committee (IACUC) of Sungkyunkwan University.

### 5-3. Evaluation of Antitumor Efficacy of IO101L via Intraperitoneal Injection Using Triple-Negative Breast Cancer Orthotopic Animal Model

### 5-3-1. Construction of Triple-Negative Breast Cancer Orthotopic Animal Model (Tumor Resection Model) and Administration of Test Substances thereto

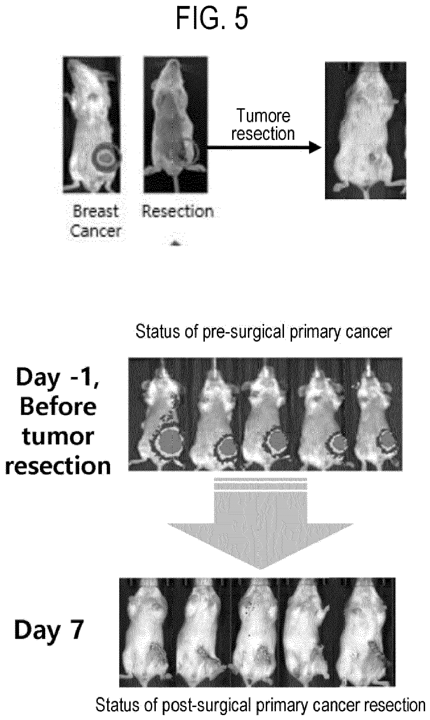

4T1-Luc2 cells (5 X 10⁵ cells), previously cultured using the described methods, were diluted in 30 µl of 1X PBS (Hyclone) (5 X 10⁵ cells/30 µl per mouse) and orthotopically injected into the 4th fat pad of the prepared female Balb/c mice. Ten days post-cell injection (when tumors reached an average size of 60mm3), the tumors were surgically removed. This process of model construction is depicted in FIG. 5. As can be seen in FIG. 5, the mice initially had primary tumors which were, however, removed post-surgery.

After tumor resection, the positive control drug gemcitabine, the negative control IO101L-Control, and the test substance IO101L were intraperitoneally (IP) injected and compared for inhibitory efficacy against tumor metastasis.

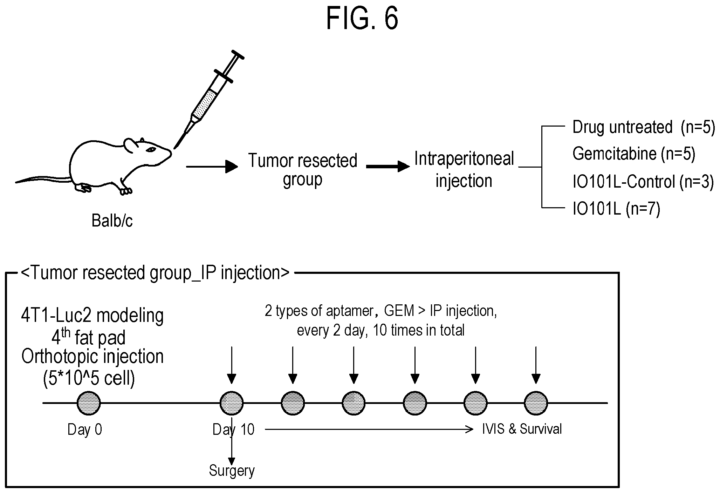

The composition of the experimental groups is summarized in Table 8, and the experimental plan is illustrated in FIG. 6. The untreated group refers to mice that underwent tumor resection without any subsequent drug injection. The positive control drug gemcitabine (1 mg), the negative control IO101L-Control (2 mg), and the test substance IO101L (2 mg) were intraperitoneally injected every other day for a total of 10 times (Day 0, 2, 4, 6, 8, 10, 12, 14, 16, 18), and the metastasis status of each group was monitored using IVIS (IVIS Lumina XRMS, Perkin Elmer).

**TABLE 8**

| **Group** | **Test substance** | **No. of animal** | **Administration method** | **Dose (mg/head)** | **No. of dosing** |
|---|---|---|---|---|---|
| G1 | Drug untreated | 5 | - | - | - |
| G2 | Gemcitabine | 5 | Intraperitoneal | 1.0 | 10 |
| G3 | IO101L-Control | 3 | Intraperitoneal | 2.0 | 10 |
| G4 | IO101L | 7 | Intraperitoneal | 2.0 | 10 |

### 5-3-2. Post-administration Observation and Measurement of Outcomes in Mice

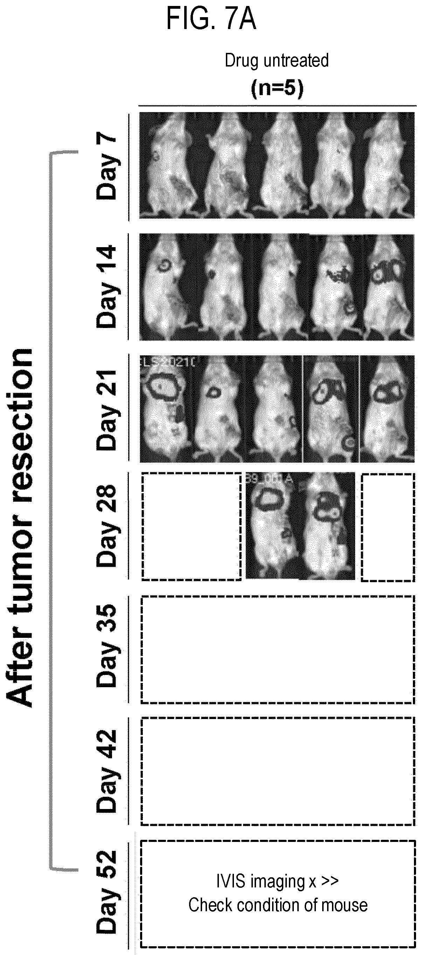

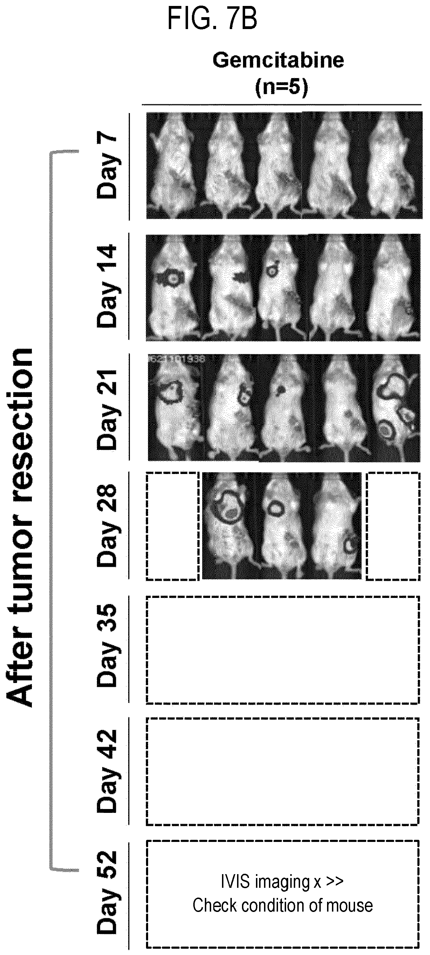

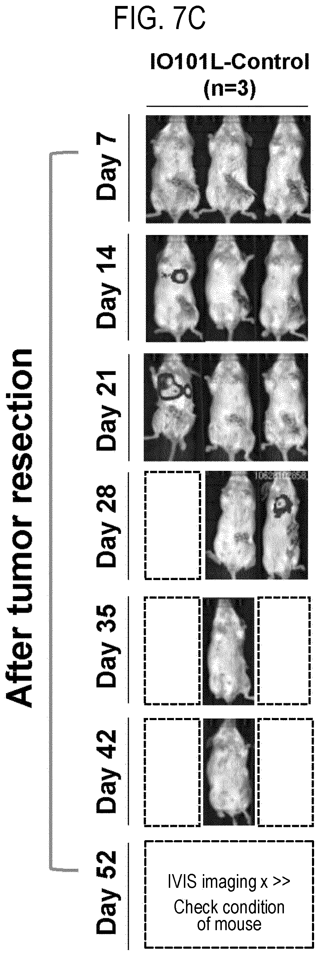

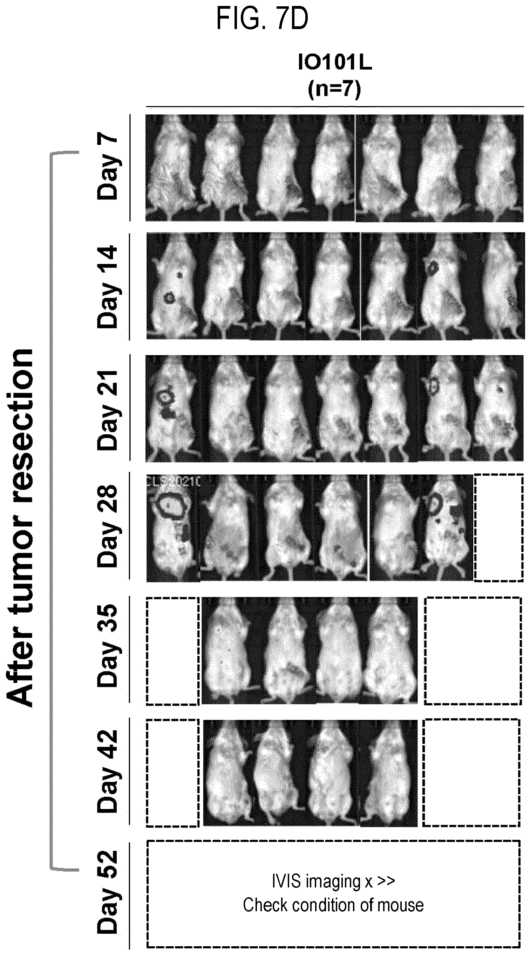

After intraperitoneal administration of the drugs, the mice were imaged using the in vivo imaging system (IVIS) every approximately 7 days until day 50. Each mouse was injected with luciferin at a concentration of 150mg/kg, and after a 10-minute reaction time, imaging was conducted using IVIS. These results are depicted in FIG. 7.

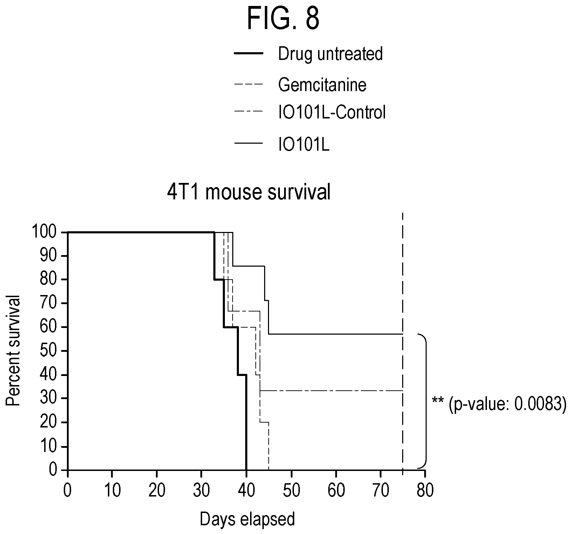

Additionally, the survival rate of mice was observed for approximately 70 days post-drug administration. The survival rates were plotted using GraphPad Prism7 program, and the p-value for survival was calculated using the Log-rank test (same as below). These results are shown in FIG. 8.

### 5-3-3. Experimental Results

In the untreated group, where no treatment was administered post-tumor removal, metastasis to the lungs was confirmed after the removal of TNBC tumors, and all subjects died within 35 days of tumor removal. Typically, mice in the untreated group succumb to cancer metastasis within a month. In the positive control group administered with gemcitabine, all mice also died within 30 days due to lung metastasis. In the negative control group administered with IO101L-Control, only one mouse survived until day 76. In the case of intraperitoneal administration of IO101L from this disclosure, lung metastasis was confirmed in only 3 out of 7 mice, and 4 out of 7 mice survived without lung metastasis until day 76. This indicates that IO101L, when administered intraperitoneally post-TNBC tumor resection, significantly inhibited cancer metastasis compared to the positive control drug, gemcitabine. It is noted that no current drug administered intraperitoneally has shown a 60% survival rate in a TNBC mouse model.

### 5-4. Evaluation of Antitumor Efficacy of IO101L-Loaded Collagen Patch Using Triple-Negative Breast Cancer Orthotopic Animal Model

### 5-4-1. Construction of Triple-Negative Breast Cancer Orthotopic Animal Model (Tumor Resection Model) and Administration of Test Substances thereto

The triple-negative breast cancer orthotopic animal model (tumor resection model) was constructed in the same manner as in 5-3-1. Ten days post-cell injection (when tumors reached an average size of 60mm³), tumors were surgically removed, and patches were implanted at the resection site to compare the efficacy in inhibiting tumor metastasis.

The composition of the experimental groups is summarized in Table 9. The untreated group refers to mice that underwent tumor resection without any subsequent treatment. The metastatic status of each group was monitored using IVIS (IVIS Lumina XRMS, Perkin Elmer) starting 10 days post-patch implantation.

**TABLE 9**

| **Group** | **Test substance** | **No. of animal** | **Administration method** | **Dose (mg/head)** |
|---|---|---|---|---|
| G1 | Drug untreated | 4 | - | - |
| G2 | 2.0 mg IO101L- loaded collagen patch | 9 | Patch implantation | 2.0 |

### 5-4-2. Post-implantation Observation and Measurement of Outcomes in Mice

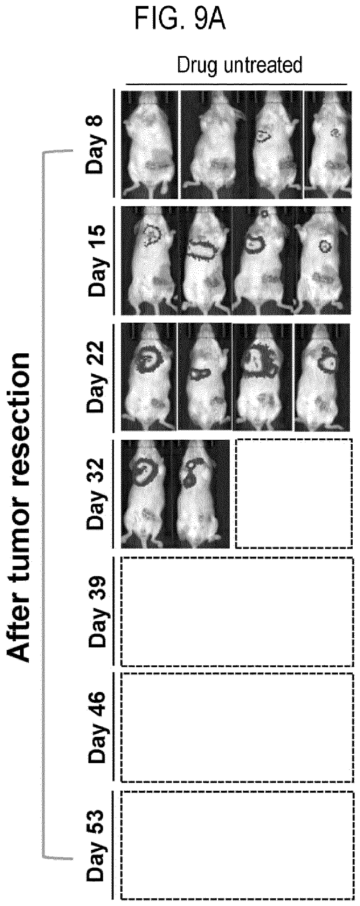

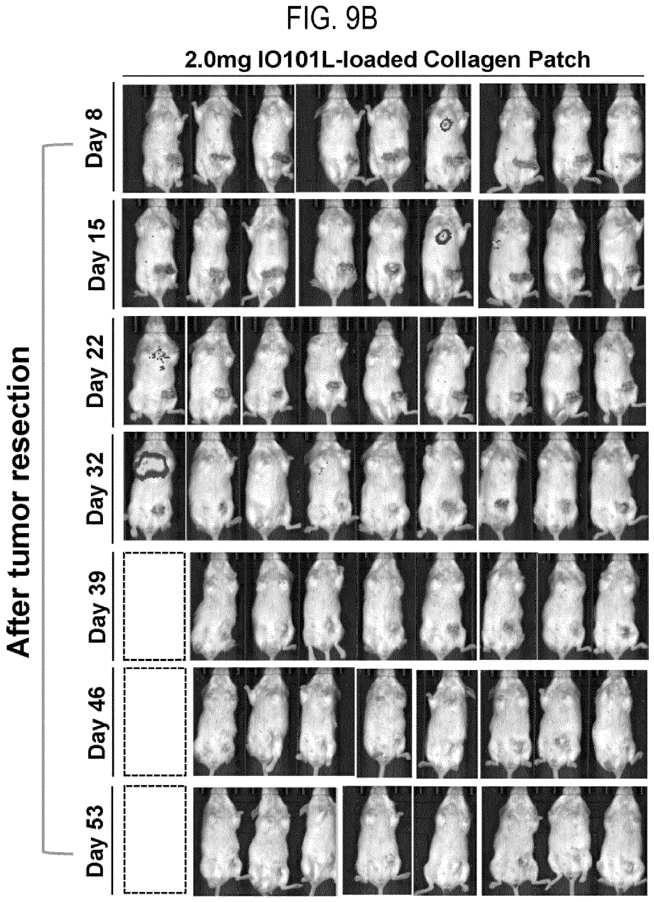

After the implantation of the patches, the mice were imaged using the in vivo imaging system (IVIS) approximately every 7 days until day 50. Each mouse was injected with luciferin at a concentration of 150mg/kg, and after a 10-minute reaction time, imaging was conducted using IVIS. The results are presented in FIG. 9.

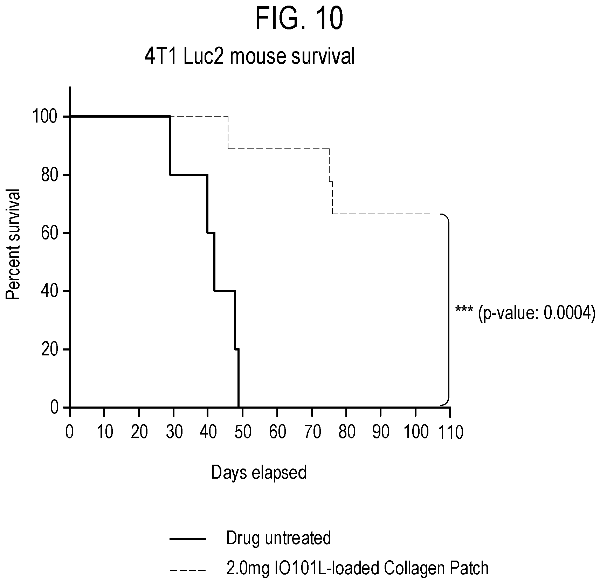

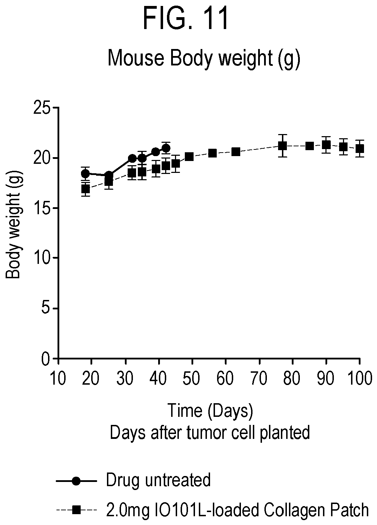

Additionally, the survival rates and weight changes of the mice were observed for approximately 90 days. The results are shown in FIGS. 10 and 11.

### 5-4-3. Experimental Results

In the untreated group, where no treatment was administered post-tumor removal, all mice succumbed to metastatic cancer cells within approximately 30 days. Typically, mice in the untreated group succumb to cancer metastasis within a month. In the experimental group implanted with IO101L-loaded collagen patches, only 1 out of 9 mice exhibited lung metastasis and died after 30 days, while the remaining 8 mice survived without any metastasis for over 90 days. These results imply that the modified oligonucleotide disclosed here effectively inhibits cancer metastasis in subjects that have undergone surgical resection of TNBC.

### 5-5. Evaluation of Dose-Dependent Antitumor Efficacy of IO101L-Loaded Collagen Patches Using Triple-Negative Breast Cancer Orthotopic Animal Model

### 5-5-1. Construction of Triple-Negative Breast Cancer Orthotopic Animal Model (Tumor Resection Model) and Administration of Test Substances thereto

The method for constructing the triple-negative breast cancer orthotopic animal model (tumor resection model) and the route of administration of the test substances are the same as described in 5-4-1.

The composition of the experimental groups is summarized in Table 10. The untreated group refers to mice that underwent tumor resection without any subsequent treatment. The metastatic status of each group was monitored using IVIS (IVIS Lumina XRMS, Perkin Elmer) starting 10 days post-patch implantation.

**TABLE 10**

| **Group** | **Test substance** | **No. of animal** | **Administration method** | **Dose (mg/head)** |
|---|---|---|---|---|
| G1 | Drug untreated | 5 | - | - |
| G2 | Collagen patch (Only Collagen Patch) | 5 | Patch implantation | - |
| G3 | Gemcitabine-loaded collagen patch | 5 | Patch implantation | 0.12 |
| G4 | 0.5mg IO101L- loaded collagen patch | 5 | Patch implantation | 0.5 |
| G5 | 2.0mg IO101L- loaded collagen patch | 5 | Patch implantation | 2.0 |
| G6 | 4.0mg IO101L- loaded collagen patch | 5 | Patch implantation | 4.0 |

### 5-5-2. Post-implantation Observation and Measurement of Outcomes in Mice

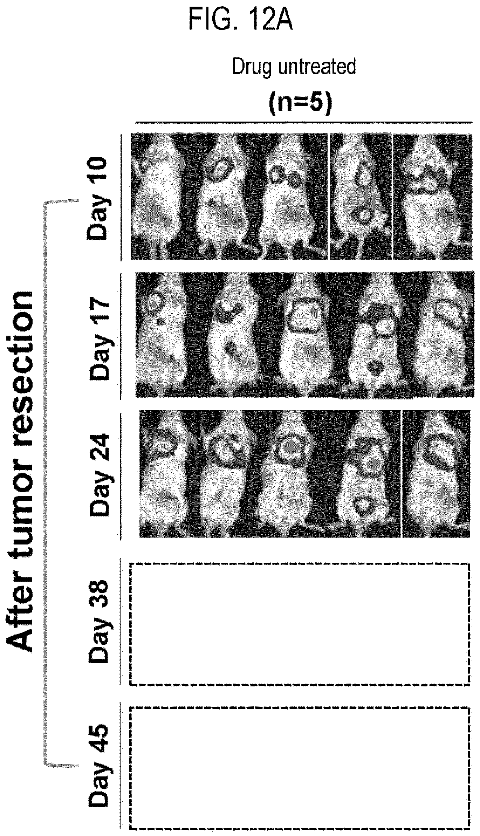

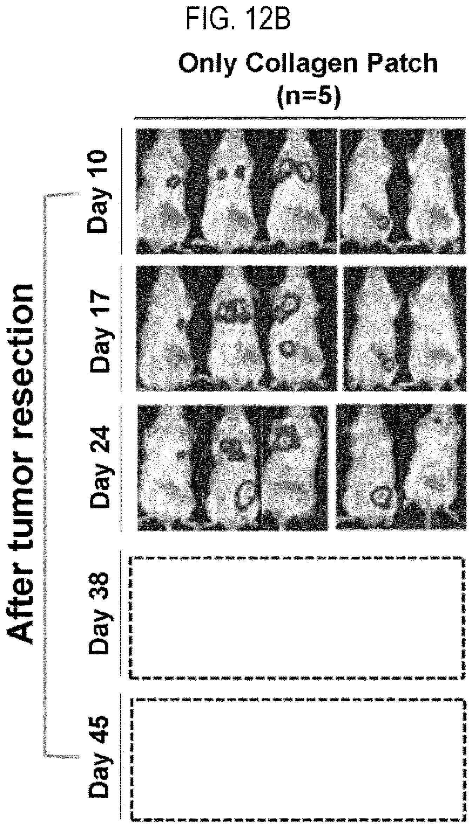

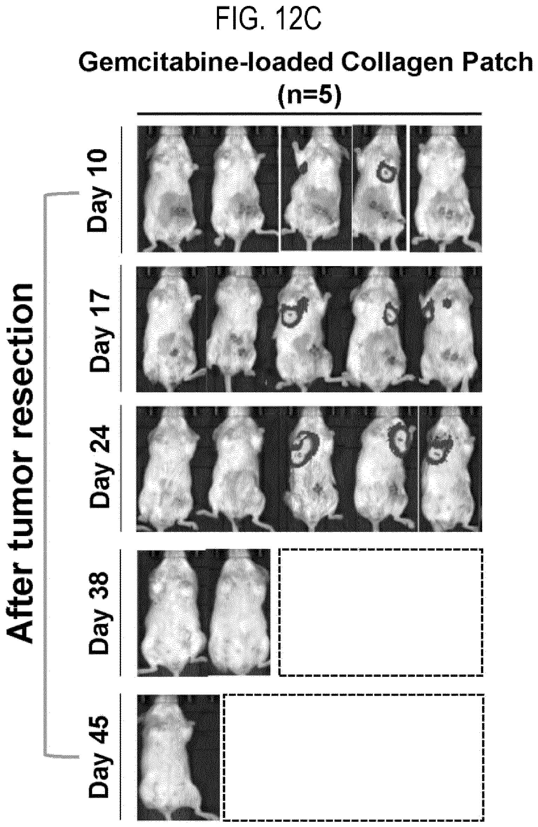

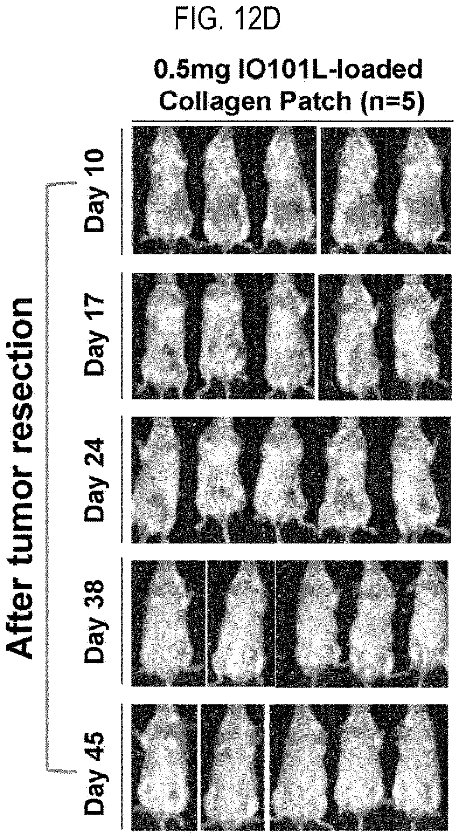

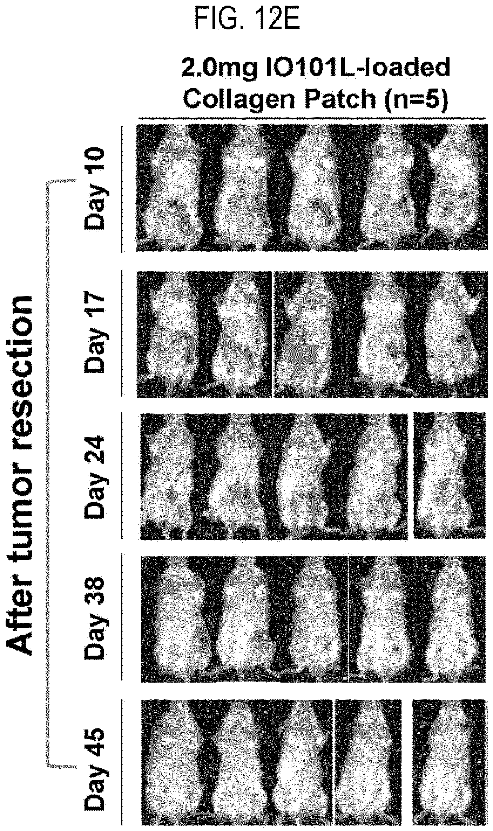

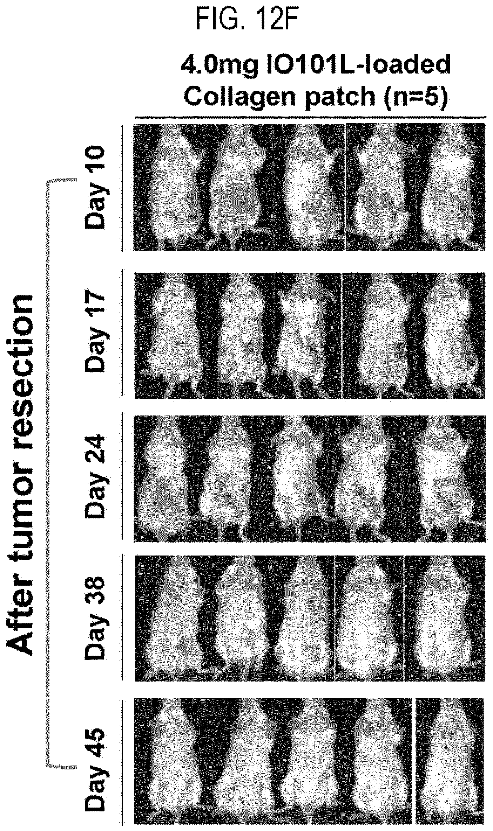

After implanting the patches, the mice were imaged using the in vivo imaging system (IVIS) approximately every 7 days for up to 50 days. Each mouse was injected with luciferin at a concentration of 150mg/kg, followed by IVIS imaging after a 10-minute reaction period. The results are displayed in FIG. 12.

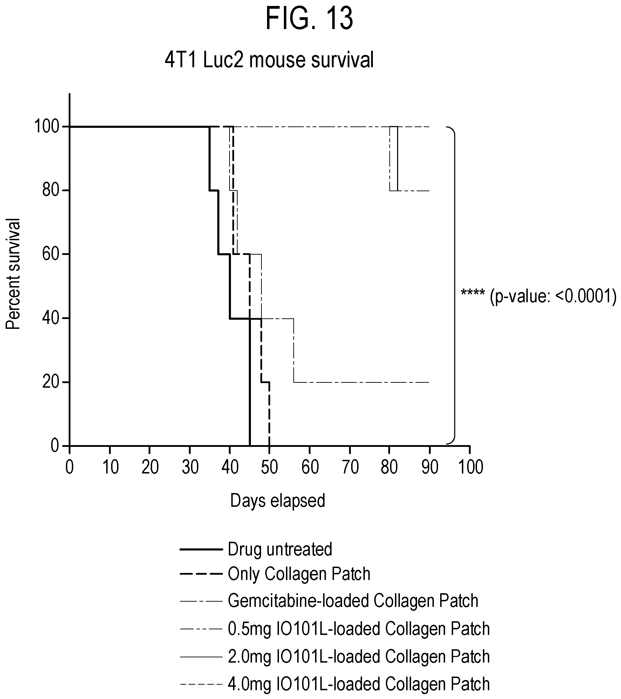

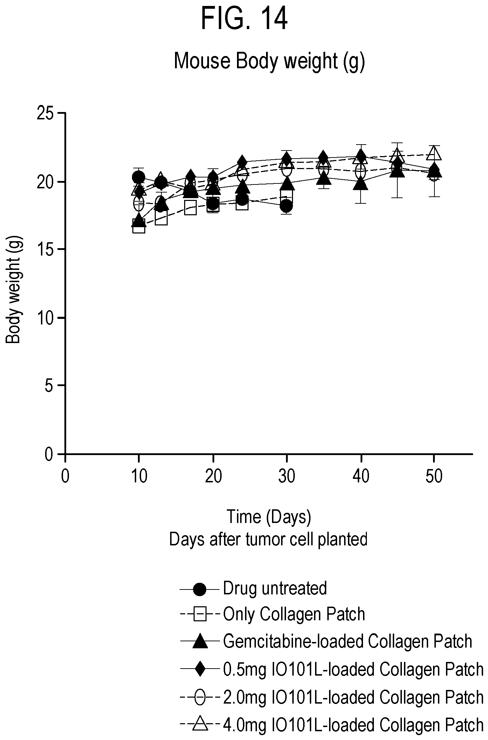

The survival rates and weight changes of the mice were observed for approximately 90 days, with results shown in FIGS. 13 and 14.

### 5-5-3. Experimental Results

In the untreated control group where no treatment was administered post-tumor removal, and the test group implanted with collagen patches lacking any drug (Only Collagen Patch), all mice succumbed to metastatic cancer within about 30 days. The group implanted with Gemcitabine-loaded collagen patches (positive control) survived until day 30, but 3 out of 5 mice showed lung metastasis and all succumbed by day 38, with only one mouse surviving until day 45. In contrast, all mice implanted with IO101L-loaded collagen patches at different doses (0.5mg, 2.0mg, and 4.0mg) survived. The 100% survival rate observed was unexpected and indicates a significantly better effect in inhibiting cancer metastasis compared to the Gemcitabine-loaded collagen patches. No existing drug has shown a 100% survival rate in TNBC mouse models. These results confirm the effective inhibition of cancer metastasis by the modified oligonucleotide in subjects having undergone surgical resection of TNBC.

The weight of the mice in the experimental groups did not significantly change over time, indicating no adverse effects from the implantation of IO101L-loaded collagen patches.

### 5-6. Evaluation of Antitumor Efficacy of IO101L-Loaded Hydrogel Patches Using Triple-Negative Breast Cancer Orthotopic Animal Model

### 5-6-1. Construction of Triple-Negative Breast Cancer Orthotopic Animal Model (Tumor Resection Model) and Administration of Test Substances thereto

The construction method and test substance administration route for the triple-negative breast cancer orthotopic animal model (tumor resection model) are the same as described in 5-4-1.

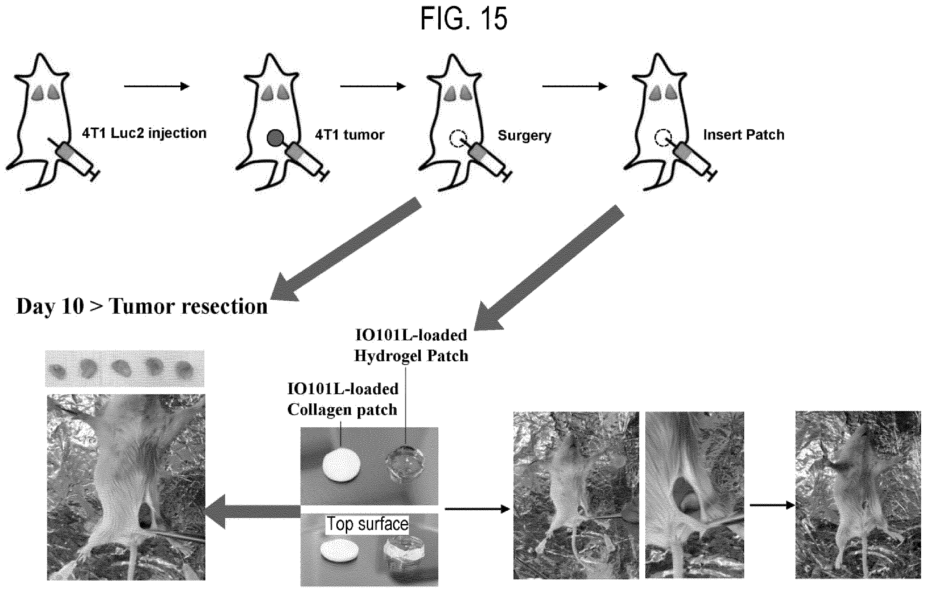

IO101L-loaded hydrogel patches were manufactured using the Hystem kit (GS310, Advanced Biomatrix, USA). Glucosil^{®} (GS222) containing 1ml DG water (Degassed, deionized water at pH 5.0-8.0) was filled into a circular mold, followed by adding 20 µl of IO101L (1 mg/20 µl) and finally, 0.25 ml of DG water containing Extralink-Lite (GS3009). The mixture was left at room temperature for approximately 90 minutes to complete gelation before implanting in the mice as described for the IO101L-loaded collagen patch in 6-5. The experimental method is illustrated in FIG. 15.

The composition of experimental groups are summarized in Table 11. The untreated group refers to mice that underwent tumor resection without any subsequent treatment. The metastatic status of each group was monitored using IVIS (IVIS Lumina XRMS, Perkin Elmer) starting 10 days post-patch implantation.

**TABLE 11**

| **Group** | **Test substance** | **No. of animal** | **Administration method** | **Dose (mg/head)** |
|---|---|---|---|---|
| G1 | Drug untreated | 5 | - | - |
| G2 | IO101L-loaded hydrogel patch | 5 | Patch implantation | 1.0 |

### 5-6-2. Post-patch implantation Observation and Measurement of Outcomes in Mice

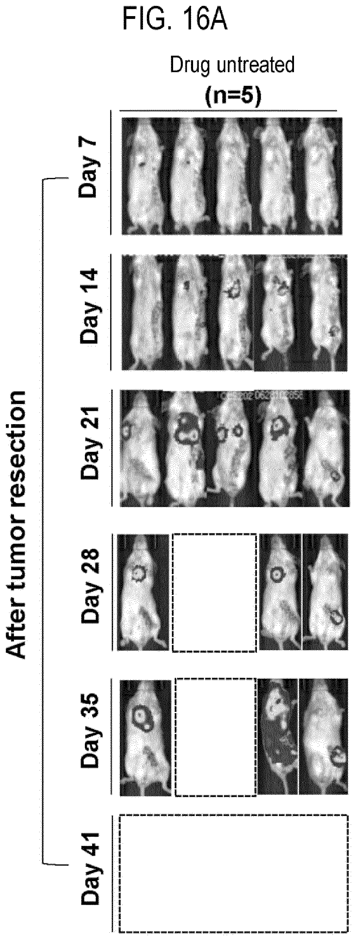

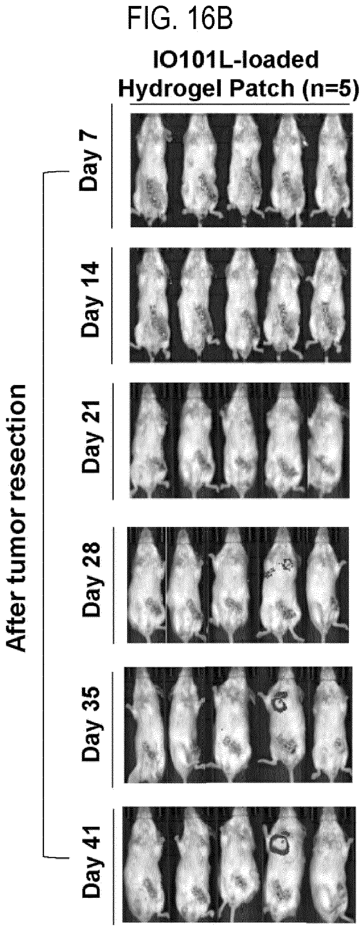

After implantation of the patches, the mice were imaged using the IVIS approximately every 7 days for up to 50 days. The IVIS imaging was performed after injecting each mouse with luciferin at a concentration of 150mg/kg and allowing a 10-minute reaction time. The results are depicted in FIG. 16.

### 5-6-3. Experimental Results

In the group untreated after tumor resection, all mice succumbed to metastatic cancer cells within approximately 41 days. However, in the group implanted with IO101L-loaded hydrogel patches, all mice were alive until day 41, with one mouse succumbing on day 48, and the remaining 4 mice surviving until day 80. These results confirm that the modified oligonucleotide effectively inhibits cancer metastasis in subjects who have undergone surgical resection of TNBC.

### [Formulation Example 1] IO101L/Collagen Sol-Gel Type Formulation

(Gem)₂-[TGG]₄[TTG][TGG]₅(IO101L) was dissolved in PBS at room temperature using a mixer until fully dissolved. It was then mixed with varying concentrations of high purity collagen dispersion (COLTRIX^{®} Tendoregen, provided by U-BioSys) at 0.5%, 1.0%, 1.5%, 2.0%, and 3.0%, combined with the PBS-mixed IO101L (e.g., 0.5mg, 2.0mg, 4.0mg based on dosage).

The mixture was blended for 30 minutes at room temperature using a multi-mixer to create the mixed solution. The (Gem)₂-[TGG]₄[TTG][TGG]₅(IO101L)/collagen (Sol-Gel Type), which remains in a liquid state (Sol) at low temperature, solidifies (Gel) at 37°C upon direct injection into the tumor in vivo. As the collagen encapsulating (Gem)₂-[TGG]₄[TTG][TGG]₅ (IO101L) gradually dissolves, the drug is slowly released, making it an effective treatment for tumors.

Although the technical spirit of the present disclosure has been described by the examples described in some embodiments and illustrated in the accompanying drawings, it should be noted that various substitutions, modifications, and changes can be made without departing from the scope of the present disclosure which can be understood by those skilled in the art to which the present disclosure pertains. In addition, it should be noted that that such substitutions, modifications and changes are intended to fall within the scope of the appended claims.

## Claims

1. A pharmaceutical composition for preventing or treating triple-negative breast cancer, comprising a modified oligonucleotide having a structure represented by Chemical Formula 1:
[Chemical Formula 1] (N)ₓ-[TGG]ₘ[TTG][TGG]ₙ-(M)_{y}
or a pharmaceutically acceptable salt thereof,
wherein N and M independently are deoxyuridine (dU), deoxycytidine (dC), uridine (U), or
cytidine (C) bound with a halogen or hydroxyl at the 5-position or 2'-position; x and y are independently integers from 0 to 10, provided that x and y are not both 0; n is an integer from 1 to 10; and m is an integer from 1 to 10.

2. The pharmaceutical composition of claim 1, wherein N and M are independently selected from the group consisting of 5-fluorodeoxyuridine, 5-fluorouridine, 5-fluorodeoxycytidine, 5-fluorocytidine, 5-iododeoxyuridine, 5-iodouridine, 5-iododeoxycytidine, 5-iodocytidine, cytosine arabinoside, 2',2'-difluorodeoxycytidine, capecitabine, and bromovinyldeoxyuridine.

3. The pharmaceutical composition of claim 1, wherein the modified oligonucleotide has a structure represented by any one of Chemical Formulas 2 to 34:
[Chemical Formula 2] (N)₂-[TGG]₁[TTG] [TGG]₁,
[Chemical Formula 3] (N)₂-[TGG]₁[TTG][TGG]₂,
[Chemical Formula 4] (N)₂-[TGG]₂[TTG][TGG]₁,
[Chemical Formula 5] (N)₂-[TGG]₂[TTG][TGG]₂,
[Chemical Formula 6] (N)₂-[TGG]₂[TTG][TGG]₃,
[Chemical Formula 7] (N)₂-[TGG]₃[TTG][TGG]₂,
[Chemical Formula 8] (N)₂-[TGG]₃[TTG][TGG]₃,
[Chemical Formula 9] (N)₂-[TGG]₃[TTG][TGG]₄,
[Chemical Formula 10] (N)₂-[TGG]₄[TTG][TGG]₃,
[Chemical Formula 11] (N)₂-[TGG]₄[TTG][TGG]₄,
[Chemical Formula 12] (N)₂-[TGG]₄[TTG][TGG]₅,
[Chemical Formula 13] (N)₂-[TGG]₅[TTG][TGG]₄,
[Chemical Formula 14] (N)₂-[TGG]₅[TTG][TGG]₅,
[Chemical Formula 15] (N)₂-[TGG]₅[TTG][TGG]₆,
[Chemical Formula 16] (N)₂-[TGG]₆[TTG][TGG]₅,
[Chemical Formula 17] (N)₂-[TGG]₆[TTG][TGG]₆,
[Chemical Formula 18] [TGG]₄[TTG][TGG]₄-(M)₁,
[Chemical Formula 19] [TGG]₄[TTG][TGG]₄-(M)₂,
[Chemical Formula 20] [TGG]₄[TTG][TGG]₄-(M)₃,
[Chemical Formula 21] [TGG]₄[TTG][TGG]₄-(M)₄,
[Chemical Formula 22] [TGG]₄[TTG][TGG]₄-(M)₅,
[Chemical Formula 23] [TGG]₄[TTG][TGG]₅-(M)₁,
[Chemical Formula 24] [TGG]₄[TTG][TGG]₅-(M)₂,
[Chemical Formula 25] [TGG]₄[TTG][TGG]₅-(M)₃,
[Chemical Formula 26] [TGG]₄[TTG][TGG]₅-(M)₄,
[Chemical Formula 27] [TGG]₄[TTG][TGG]₅-(M)₅,
[Chemical Formula 28] [TGG]₄[TTG][TGG]₄-(M)₁₀,
[Chemical Formula 29] (N)₁-[TGG]₄[TTG][TGG]₄-(M)₁,
[Chemical Formula 30] (N)₃-[TGG]₄[TTG][TGG]₄-(M)₃,
[Chemical Formula 31] (N)₅-[TGG]₄[TTG][TGG]₄-(M)₅,
[Chemical Formula 32] (N)₁-[TGG]₄[TTG][TGG]₅-(M)₁,
[Chemical Formula 33] (N)₃-[TGG]₄[TTG][TGG]₅-(M)₃,
[Chemical Formula 34] (N)₅-[TGG]₄[TTG][TGG]₅-(M)₅

4. The pharmaceutical composition of claim 1, wherein n is an integer from 1 to 5, and m is an integer from 1 to 5.

5. The pharmaceutical composition of claim 1, wherein x and y are independently integers from 0 to 5, provided that x and y are not both 0.

6. The pharmaceutical composition of claim 1, wherein the modified oligonucleotide has a structure represented by Chemical Formula 12:
[Chemical Formula 12] (N)₂-[TGG]₄[TTG][TGG]₅.

7. The pharmaceutical composition of claim 6, wherein N is 2',2'-difluorodeoxycytidine.

8. The pharmaceutical composition of claim 1, wherein the triple-negative breast cancer is a metastatic triple-negative breast cancer.

9. The pharmaceutical composition of claim 1, wherein the pharmaceutical composition is administered to a subject who has undergone surgical resection of triple-negative breast cancer.

10. The pharmaceutical composition of claim 9, wherein the pharmaceutical composition prevents or treats metastatic cancer or tumors in the subject.

11. The pharmaceutical composition of claim 10, wherein the metastatic cancer is lung cancer, brain cancer, osteosarcoma, liver cancer, or lymphoma.

12. The pharmaceutical composition of claim 1, wherein the modified oligonucleotide is administered at a dose of about 0.01 mg/kg to about 100 mg/kg.

13. The pharmaceutical composition of claim 1, wherein the pharmaceutical composition is administered once a week, once every two weeks, once every three weeks, or once a month.

14. The pharmaceutical composition of claim 1, wherein the pharmaceutical composition further comprises a biomaterial.

15. The pharmaceutical composition of claim 1, wherein the pharmaceutical composition is administered in a formulation of a collagen dispersion or a hydrogel dispersion.

16. The pharmaceutical composition of claim 1, wherein the pharmaceutical composition is administered in a formulation of a sol-gel or patch form.

17. The pharmaceutical composition of claim 1, wherein the pharmaceutical composition is implanted at a site of surgical resection of triple-negative breast cancer in a subject in need thereof.

18. The pharmaceutical composition of claim 1, wherein the pharmaceutical composition is administered intravenously, intramuscularly, intra-arterially, intraperitoneally, intranasally, intravaginally, intravesically, intradermally, transdermally, topically, or subcutaneously to a subject in need thereof.

19. A pharmaceutical composition for preventing or treating metastatic cancer or tumors occuring after surgical resection of triple-negative breast cancer, comprising a modified oligonucleotide having a structure represented by Chemical Formula 1:
[Chemical Formula 1] (N)ₓ-[TGG]ₘ[TTG][TGG]ₙ-(M)_{y},
or a pharmaceutically acceptable salt thereof,
wherein the pharmaceutical composition is administered to a subject who has undergone surgical resection of triple-negative breast cancer,
wherein N and M independently are deoxyuridine (dU), deoxycytidine (dC), uridine (U), or
cytidine (C) bound with a halogen or hydroxyl at the 5-position or 2'-position; x and y are independently integers from 0 to 10, provided that x and y are not both 0; n is an integer from 1 to 10; and m is an integer from 1 to 10.
